# EUROPEAN PATENT APPLICATION

(11) **EP 4 809 904 A1**
(43) Date of publication of application: **23.09.2026**
(21) Application number: 24891059.8
(22) Date of filing: 02.09.2024
(51) Int. Cl.: A61B 1/018

(54) **ENDOSCOPE**

(30) Priority: 15.11.2023 US 202363599292 P
(71) Applicant: Olympus Medical Systems Corp., Tokyo 192-8507 (JP)
(72) Inventor: SEKI, Hiroyuki, Hachioji-shi, Tokyo 192-8507 (JP); HIRASAWA, Yoshihiro, Hachioji-shi, Tokyo 192-8507 (JP); NEMOTO, Mitsutaka, Hachioji-shi, Tokyo 192-8507 (JP); SAKIYAMA, Masakazu, Hachioji-shi, Tokyo 192-8507 (JP); ISHIOKA, Takaki, Hachioji-shi, Tokyo 192-8507 (JP); MITA, Kentaro, Hachioji-shi, Tokyo 192-8507 (JP); MORI, Kotaro, Hachioji-shi, Tokyo 192-8507 (JP); DOI, Daisuke, Hachioji-shi, Tokyo 192-8507 (JP); IGUCHI, Kento, Hachioji-shi, Tokyo 192-8507 (JP)
(74) Representative: Schicker, Silvia
(86) International application number: PCT/JP2024/031490
(87) International publication number: WO 2025/105016

(57) **Abstract**

An endoscope (1) includes a flexible tube (5) along a center axis (O). The flexible tube (5) includes, on an outer circumferential surface thereof, a plurality of protruded surfaces (6) and recessed surfaces (7). The plurality of protruded surfaces (6) are each surrounded by the recessed surfaces (7) to be isolated from one another. When the recessed surfaces (7) each include a first part (7c1) along an axial direction parallel to the center axis (O), the first parts (7c1) are formed intermittently in the axial direction. When the recessed surface (7) each include a second part (7c2) along a circumferential direction around the center axis (O), the second parts (7c2) are formed intermittently in the circumferential direction.

## Description

### Technical Field

The present disclosure relates to an endoscope including a flexible tube having flexibility that is formed in a tubular shape.

### Background Art

As examples of flexible tubes, corrugated tubes have been known conventionally. Corrugated tubes include a bellows structure in which ring-shaped protruded portions around an axis and ring-shaped recessed portions around the axis are alternately formed along a center axis in a longitudinal direction. Corrugated tubes can be manufactured inexpensively, have a characteristic of high bendability, and are used for a covering material of an electric wire, for example.

Incidentally, flexible endoscopes include an insertion portion configured as a tube having flexibility. In recent years, single-use endoscopes, which are disposed of after a single use, have been proposed. Such single-use endoscopes can preferably be manufactured inexpensively in view of costs, and a use of corrugated tubes are considered.

Japanese Patent Application Laid-Open Publication No. H09-066021, for example, recites an endoscope provided with a bending portion having an inner tube configured in a bellows shape.

In general, characteristics required for an insertion portion of an endoscope include bendability, an extension/contraction resistance, and a torque resistance. The bendability is, as is well known, bending performance. The extension/contraction resistance is a resistance to extension and contraction. Specifically, the extension/contraction resistance includes a compression resistance which is a resistance to compression and a tension resistance which is a resistance to tension. In addition, the torque resistance is a resistance to twisting around a center axis.

A corrugated tube having a bellows structure has a bendability and a high torque resistance. However, the corrugated tube has a low extension/contraction resistance, and contracts upon receipt of a compression force and extends upon receipt of a pulling force.

The above-described several characteristics are in a trade-off relationship with one another in many cases. For this reason, it is difficult to increase the extension/contraction resistance and the torque resistance of the corrugated tube having the bellows structure, while maintaining the bendability thereof. In addition, in the corrugated tube having the bellows structure, it is also difficult to more freely control the characteristics such as viscoelasticity at the time of bending.

The present disclosure is achieved in view of the above-described circumstances, and an object of the present disclosure is to provide an endoscope including a flexible tube that can be manufactured inexpensively, while balancing a bendability, an extension/contraction resistance, and a torque resistance thereof.

### Disclosure of Invention

### Means for Solving the Problem

An endoscope according to one aspect of the present disclosure includes a flexible tube. The flexible tube is a tube that is along a center axis extending from one end to another end, and the flexible tube includes: a plurality of protruded surfaces provided on an outer circumferential surface of the flexible tube; and recessed surfaces provided on the outer circumferential surface, the recessed surfaces being defined by the plurality of protruded surfaces, the plurality of protruded surfaces are each surrounded by the recessed surfaces, so as to be isolated from one another, when the recessed surfaces each include a first part along an axial direction parallel to the center axis, the first parts are formed intermittently in the axial direction, and when the recessed surfaces each include a second part along a circumferential direction around the center axis, the second parts are formed intermittently in the circumferential direction.

An endoscope according to one aspect of the present disclosure includes a flexible tube. The flexible tube is a tube that is along a center axis extending from one end to another end and includes: a plurality of protruded surfaces provided on an outer circumferential surface of the flexible tube; and recessed surfaces provided on the outer circumferential surface, the recessed surfaces being defined by the plurality of protruded surfaces. The plurality of protruded surfaces are each surrounded by the recessed surfaces, so as to be isolated from one another, and the recessed surfaces are all inclined with respect to an axial direction parallel to the center axis, and inclined with respect to a circumferential direction around the center axis.

### Brief Description of the Drawings

FIG. 1 shows a configuration example of an endoscope in a first embodiment of the present disclosure.
FIG. 2 is a perspective view showing an example of a flexible tube in the first embodiment.
FIG. 3 is a perspective view showing a modified example of the flexible tube in the first embodiment.
FIG. 4 shows a configuration, in a planarly developed state, of an outer circumferential surface of the flexible tube in the first embodiment.
FIG. 5 shows a configuration, in a planarly developed state, of an outer circumferential surface of a flexible tube in a second embodiment of the present disclosure.
FIG. 6 is a perspective view showing a flexible tube in a third embodiment of the present disclosure.
FIG. 7 shows a configuration, in a planarly developed state, of an outer circumferential surface of the flexible tube in the third embodiment.
FIG. 8 shows a configuration, in a planarly developed state, of an outer circumferential surface of a flexible tube in a fourth embodiment of the present disclosure.
FIG. 9 shows a configuration, in a planarly developed state, of an outer circumferential surface of a flexible tube in a fifth embodiment of the present disclosure.
FIG. 10 shows a configuration, in a planarly developed state, of an outer circumferential surface of a flexible tube in a sixth embodiment of the present disclosure.
FIG. 11 shows a configuration, in a planarly developed state, of an outer circumferential surface of a flexible tube in a seventh embodiment of the present disclosure.
FIG. 12 shows a configuration, in a planarly developed state, of an outer circumferential surface of a flexible tube in an eighth embodiment of the present disclosure.
FIG. 13 shows a configuration, in a planarly developed state, of an outer circumferential surface of a flexible tube in a ninth embodiment of the present disclosure.
FIG. 14 shows a configuration, in a planarly developed state, of an outer circumferential surface of a flexible tube in a tenth embodiment of the present disclosure.
FIG. 15 is a perspective view showing a flexible tube in an eleventh embodiment of the present disclosure.
FIG. 16 is a perspective view showing a flexible tube in a twelfth embodiment of the present disclosure.
FIG. 17 is a perspective view showing a flexible tube in a thirteenth embodiment of the present disclosure.
FIG. 18 is a cross-sectional view showing a first connecting structure of a tube and a rigid member in a related technology.
FIG. 19 is a cross-sectional view showing a second connecting structure of the tube and the rigid member in the related technology.
FIG. 20 is a cross-sectional view showing a third connecting structure of the tube and the rigid member in the related technology.
FIG. 21 is a cross-sectional view showing a fourth connecting structure of the tube and the rigid member in the related technology.
FIG. 22 is a cross-sectional view showing a fifth connecting structure of the tube and the rigid member in the related technology.
FIG. 23 is a cross-sectional view showing a sixth connecting structure of the tube and the rigid member in the related technology.
FIG. 24 is a cross-sectional view showing a seventh connecting structure of the tube and the rigid member in the related technology.
FIG. 25 is a cross-sectional view showing an eighth connecting structure of the tube and the rigid member in the related technology.
FIG. 26 is a chart describing a manufacturing method of the tube according to a ninth connecting structure of the tube and the rigid member in the related technology.

### Best Mode for Carrying Out the Invention

Hereinafter, embodiments of the present disclosure will be described with reference to drawings. However, the present disclosure is not limited by the embodiments to be described below.

Note that in the illustrations in the drawings, the same or corresponding elements are attached with the same reference signs as appropriate. In addition, the drawings are schematic, and care should be taken to the fact that a relationship among the lengths of the respective elements, a ratio of the lengths of the respective elements, the number of the respective elements, and the like are sometimes different from the actual ones for simplification of the description. Furthermore, the respective drawings sometimes include parts in which the relationships and ratios among the lengths of the elements, the number of the elements, and the like are different.

### [First Embodiment]

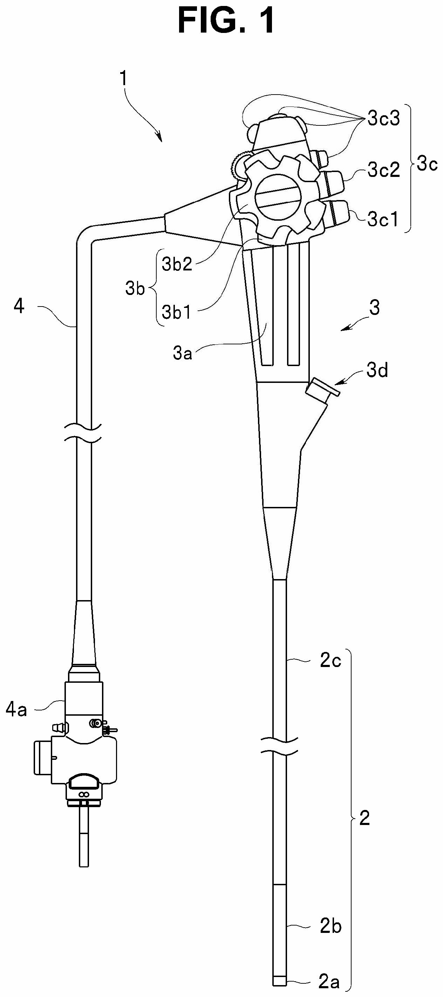

FIG. 1 to FIG. 4 each show a first embodiment of the present disclosure. FIG. 1 shows a configuration example of an endoscope 1 in the first embodiment.

The endoscope 1 is a device configured to perform observation and treatment of a subject. The endoscope 1 includes an insertion portion 2 configured to be inserted into the subject, an operation portion 3 provided on a proximal end side of the insertion portion 2, and a universal cord 4 extended from the operation portion 3.

Note that the subject into which the insertion portion 2 is inserted is supposed to be a living thing such as a human being or an animal, but not limited thereto. The subject may be a non-living thing such as a machine or architecture. In addition, the endoscope 1 may be an automatically inserting endoscope configured to advance or retreat by rotation. The endoscope 1 may be any of an upper gastrointestinal endoscope, a lower gastrointestinal endoscope, or an endoscope to be applied to other parts.

The insertion portion 2 includes, in the following order from the distal end toward the proximal end, a distal end portion 2a, a bending portion 2b, and a tubular portion 2c.

The distal end portion 2a includes an observation system and an illumination system, for example. The illumination system includes an illumination optical system and the like, and applies illumination light to a subject. The observation system includes, inside an observation window, an objective optical system and an image pickup device. The observation system forms an image of return light from the subject by the objective optical system and picks up the image by the image pickup device.

The bending portion 2b is provided on the proximal end side of the distal end portion 2a, and configured to be bendable in two directions, i.e., up and down directions, or in four directions, i.e., up, down, left, and right directions, for example. When the bending portion 2b is bent, a direction of the distal end portion 2a is changed, and a direction of observation by the observation system and an irradiation direction of illumination light by the illumination system are changed. In addition, the bending portion 2b is bent also for improving insertion performance of the insertion portion 2 in the subject.

The tubular portion 2c is a tubular part connecting the proximal end of the bending portion 2b and the distal end of the operation portion 3. The tubular portion 2c has a flexible form that sags according to the shape of the subject into which the tubular portion 2c is inserted. In this case, the endoscope 1 is called a flexible endoscope.

The operation portion 3 is provided on the proximal end side of the insertion portion 2, and is a part with which various operations related to the endoscope 1 are performed by being grasped by the hand. The operation portion 3 includes, for example, a grasping portion 3a, a bending operation knob 3b, a plurality of operation buttons 3c, and a treatment instrument insertion port 3d.

The grasping portion 3a is a part for an operator to grasp the endoscope 1 with the palm.

The bending operation knob 3b is an operation device for performing bending operation of the bending portion 2b by using the thumb of the hand grasping the grasping portion 3a, for example. When the bending portion 2b is bendable in four directions, i.e., up, down, left, and right directions, the bending operation knob 3b includes a UD bending operation knob 3b1 for bending operation in the up and down directions, and an RL bending operation knob 3b2 for bending operation in the left and right directions.

The plurality of operation buttons 3c include, for example, a gas/liquid feeding button 3c1, a suction button 3c2, and other buttons 3c3, for example.

The gas/liquid feeding button 3c1 is a button for performing gas/liquid feeding to the observation window provided on the distal end surface of the observation system via a gas/liquid feeding channel, not shown, to thereby perform operation for cleaning the observation window.

The suction button 3c2 is a button for performing an operation for sucking a liquid, a mucosa, and the like from the subject via a suction channel, not shown.

The other buttons 3c3 are used as a freeze button for temporarily stopping a monitor screen, a release button for picking up a still image, a switching button for switching to special light, and the like, for example.

The treatment instrument insertion port 3d is provided on a distal-end-side lateral surface of the grasping portion 3a. The treatment instrument insertion port 3d is communicated with a treatment instrument channel. The treatment instrument channel includes a distal-end-side opening at the distal end portion 2a. When various treatment instruments such as forceps are inserted from the treatment instrument insertion port 3d, the distal end of the treatment instrument protrudes from the distal-end-side opening of the treatment instrument channel, to enable various kinds of treatment to be performed on the subject.

The universal cord 4 is extended from the proximal-end-side lateral surface of the operation portion 3, for example. The universal cord 4 includes, at the extension end thereof, a connector 4a. The connector 4a is connected to an endoscope processor and a light source apparatus (or an endoscope processor which functions also as the light source apparatus), which are not shown.

The endoscope processor is configured to transmit a driving signal and power to the image pickup device in the distal end portion 2a. In addition, the endoscope processor is configured to receive an image pickup signal obtained by picking up an image of the subject by the image pickup device. The light source apparatus emits illumination light to transmit the illumination light via a light guide not shown. The illumination light transmitted by the light guide is applied from the distal end surface of the distal end portion 2a to the subject.

Note that, instead of the configuration in which the illumination light emitted by the light source apparatus is transmitted by the light guide, a configuration may be employed in which a light-emitting element is provided in the distal end portion 2a, power is supplied from the endoscope processor to the light-emitting element, and the light-emitting element emits the illumination light.

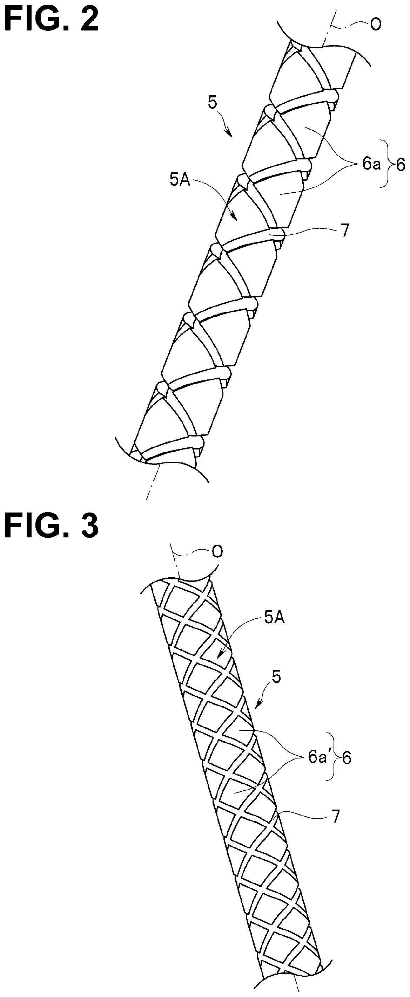

FIG. 2 is a perspective view showing an example of a flexible tube 5 in the first embodiment.

The endoscope 1 includes the flexible tube 5. The flexible tube 5 is provided, for example, in the insertion portion 2 (that is, the part including the bending portion 2b and the tubular portion 2c) of the endoscope 1. However, the configuration is not limited to the above, and the flexible tube 5 may be provided in the universal cord 4. Furthermore, the flexible tube 5 is not limited to be used for the endoscope 1, and can be widely applied to medical devices. For example, the flexible tube 5 may be applied to a treatment instrument such as a catheter, an overtube, and the like.

The flexible tube 5 is a tube having a flexibility, which is along a center axis O extending from one end to the other end. The flexible tube 5 includes, on an outer circumferential surface 5A thereof, a plurality of protruded surfaces 6 and recessed surfaces 7 defined by the plurality of protruded surfaces 6. The recessed surfaces 7 are each a bottomed surface and different from a slit.

The flexible tube 5 is formed by feeding a heated material such as plastic from an extruder into a cylindrical-shaped mold, and by making the material closely contact the inner circumferential surface of the mold by a vacuum mechanism provided in the mold. The cylindrical-shaped mold is constituted of a pair of molds which are divided into two by a plane passing through the center axis, for example. The pair of molds is used by being transported like an endless crawler, for example.

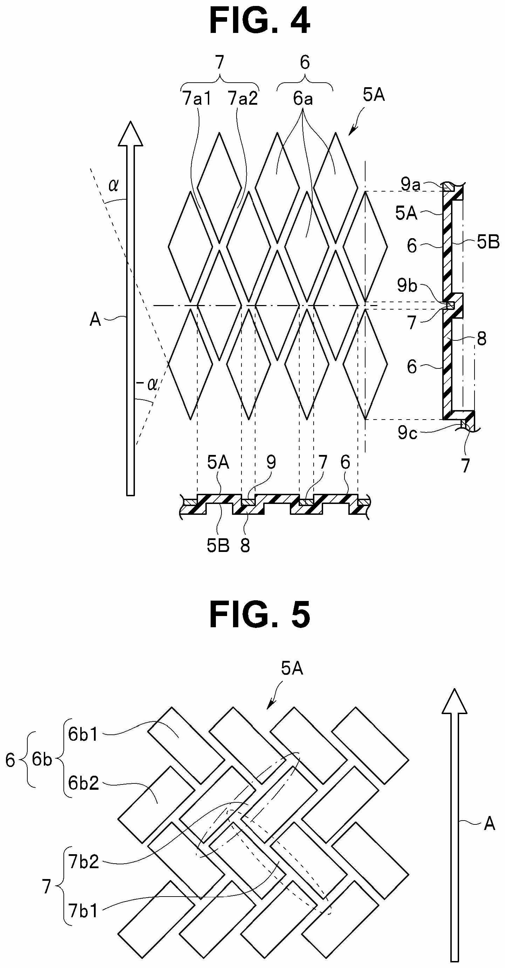

Therefore, the shapes of the protruded surfaces 6 and the recessed surfaces 7 that are formed on the outer circumferential surface 5A of the flexible tube 5 are defined accurately in accordance with the mold. On the other hand, an uneven shape of the inner circumferential surface 5B of the flexible tube 5 (see FIG. 4) is roughly formed according to the material that is made to closely contact the inner circumferential surface with a substantially even thickness. In other words, when viewing from the inner circumferential surface 5B of the flexible tube 5, the parts corresponding to the protruded surfaces 6 are recessed surfaces and the part corresponding to the recessed surfaces 7 are protruded surfaces.

The plurality of protruded surfaces 6 are each surrounded by the recessed surfaces 7, to be isolated from one another. In other words, the plurality of protruded surfaces 6 are formed intermittently in the axial direction parallel to the center axis O and in the circumferential direction around the center axis O.

The plurality of protruded surfaces 6 each have the same shape, for example, (note that description will be made later on the example in which the plurality of protruded surfaces have different shapes) and arranged periodically along the outer circumferential surface 5A of the flexible tube 5. Each of the plurality of protruded surfaces 6 forms a polygon, in particular, a quadrangle in the present embodiment, when the outer circumferential surface 5A of the flexible tube 5 is planarly developed.

Specifically, in the example shown in FIG. 2, each of the plurality of protruded surfaces 6 is a rhombus protruded surface 6a that forms a rhombus when the outer circumferential surface 5A of the flexible tube 5 is planarly developed. Note that the "rhombus protruded surface" is hereinafter just referred to as "rhombus", and the recitation of "protruded surface" will be omitted. Similarly, also protruded surfaces having other shapes, which will be described later, are recited by omitting the recitation of "protruded surfaces".

As is known, the rhombus 6a has four sides having the same length. Except for the case where the rhombus 6a is a square, one of two diagonal lines is longer than the other.

The protruded surfaces 6 and the recessed surfaces 7 are formed to be plane symmetrical with the plane passing through the center axis O, for example. In such a configuration, when molding is performed using the mold divided into two, the separation from the mold is easy.

FIG. 3 is a perspective view showing a modified example of the flexible tube 5 in the first embodiment.

When the diameter of the flexible tube 5 shown in FIG. 2 and the diameter of the flexible tube 5 shown in FIG. 3 are the same, for example, an area of a rhombus 6a´ shown in FIG. 3 is relatively smaller than an area of the rhombus 6a shown in FIG. 2. For the rhombuses 6a shown in FIG. 2, two rhombuses are arranged in the circumferential direction, while for the rhombuses 6a´ shown in FIG. 3, four rhombuses are arranged in the circumferential direction, for example. The number of the protruded surfaces 6 arranged in the circumferential direction is not limited to an even number and may be an odd number, or may be an appropriate number.

FIG. 4 shows a configuration, in a planarly developed state, of the outer circumferential surface 5A of the flexible tube 5 in the first embodiment. Note that FIG. 4 shows also a cross section in the axial direction and a cross section in the circumferential direction of the flexible tube 5 that is planarly developed.

The rhombuses 6a in the example shown in FIG. 2 are subjected to periodic tiling (however, tiling except for the recessed surfaces 7, the same applies hereafter) such that, for example, the longer diagonal line is parallel to the axial direction (direction of the arrow A shown in FIG. 4).

Then, the recessed surfaces 7 are all inclined with respect to the axial direction, and inclined with respect to the circumferential direction (direction vertical to the arrow A in the developed view in FIG. 4).

Specifically, the recessed surfaces 7 each include a first inclined part 7a1 inclined at an angle α with respect to the axial direction and a second inclined part 7a2 inclined at an angle -α with respect to the axial direction. Here, α is an angle of 45 degrees or less. The first inclined part 7a1 and the second inclined part 7a2 are spiral-shaped recessed surfaces 7 in the flexible tube 5.

Note that α is not limited to 45 degrees or less, and it may be set to an angle larger than 45 degrees as required by a design.

For example, if α is set to 45 degrees or less, compared to the case where α is set to larger than 45 degrees, the torque resistance becomes slightly low, but the extension/contraction resistance can be increased. Therefore, α may be set to 45 degrees or less in the case where an emphasis is placed on suppressing the extension or the contraction of the flexible tube 5 when the flexible tube 5 has received a compression force or a pulling force.

Conversely, when α is set to larger than 45 degrees, compared to the case where α is set to 45 degrees or less, the extension/contraction resistance becomes slightly low, but the torque resistance can be increased. Therefore, α may be set to larger than 45 degrees in the case where an emphasis is placed on suppressing the twisting of the flexible tube 5 when the flexible tube 5 has received a twisting force around the center axis O.

Thus, controlling the angle α enables control of determining a balance between the extension/contraction resistance and the torque resistance that are in the trade-off relationship with each other.

In addition, as shown in the cross section of FIG. 4, the recessed surfaces 7 may be filled with a filling material to form filling structure parts 9.

In other words, the flexible tube 5 may include a tube main body 8 and the filling structure parts 9.

The tube main body 8 is formed by using a material having a first Young's modulus so as to include the plurality of protruded surfaces 6 and the recessed surfaces 7 by the above-described mold.

The filling structure parts 9 are formed by filling the recessed surfaces 7 of the tube main body 8 with the filling material having a second Young's modulus lower than the first Young's modulus.

Further, a filling rate of the filling material in the filling structure parts 9 may be made different along the axial direction. In the example shown in the cross section in the axial direction in FIG. 4, an example is shown in which the filling rate is decreased sequentially in the axial direction in the order of a proximal-end-side filling structure part 9a, a middle filling structure part 9b, and a distal-end-side filling structure part 9c.

Note that as shown in the cross section in the circumferential direction in FIG. 4, the filling rate of the filling material in the circumferential direction is constant. However, in order to make the easiness of bending different according to the bending direction, the filling rate of the filling material in the circumferential direction may be changed.

In addition, as shown in FIG. 4, a distance from the recessed surface 7 to the protruded surface 6 in the radial direction, with the center axis O as a center (the depth of the recessed surface 7 with respect to the protruded surface 6 or the height of the protruded surface 6 with respect to the recessed surface 7) may be made different according to the position of each of the recessed surfaces 7 on the flexible tube 5. In the example shown in FIG. 4, the depth of the recessed surface 7 at a position where the filling structure part 9c is provided is set to be deeper than the depths of the respective recessed surfaces 7 at positions where the filling structure parts 9a and 9b are respectively provided. Note that the depth from the protruded surface 6 to the recessed surface 7 may be made different according to the angle of the recessed surface 7 with respect to the axial direction.

Thus, the filling structure parts 9 are provided and the depths of the recessed surfaces 7 are adjusted according to the positions thereof on the flexible tube 5, which enables the characteristics of the flexible tube 5, such as the bendability and the viscoelasticity at the time of bending to be controlled more freely.

According to the first embodiment, the flexible tube 5 has a structure including the plurality of protruded surfaces 6 each surrounded by the recessed surfaces 7 such that the plurality of protruded surfaces 6 are isolated from one another. Such a configuration enables the flexible tube 5 in the present embodiment to achieve the characteristics which cannot be achieved by the corrugated tube having the bellows structure in which ring-shaped protruded portions and ring-shaped recessed portions are alternately formed along the direction of the center axis O.

The flexible tube 5 in the present embodiment has a surface smoothness which is a little higher than that of the corrugated tube having the bellows structure. In particular, when the filling structure parts 9 are formed respectively in the recessed surfaces 7, the surface smoothness can be more enhanced, which can provide a configuration more suitable for medical devices including the endoscope 1 configured to be inserted into a subject.

The flexible tube 5 in the present embodiment includes the recessed surfaces 7 which are inclined with respect to the axial direction and inclined with respect to the circumferential direction. Such a flexible tube 5 can increase the extension/contraction resistance while maintaining the bendability.

In the flexible tube 5 in the present embodiment, the balance among the bendability, the extension/contraction resistance, and the torque resistance can be controlled more freely. Specifically, various parameters such as the angle α of the recessed surfaces 7, the depth of the recessed surfaces 7 from the protruded surfaces 6, etc., can be adjusted, thereby providing a high degree of freedom in controlling the characteristics.

The filling structure parts 9 are provided respectively in the recessed surfaces 7 by filling the recessed surfaces 7 with the filling material having the Young's modulus lower than that of the tube main body 8, and the filling rates of the filling structure parts 9 are adjusted according to the positions thereof, to thereby be capable of providing an elastic deformation characteristic different from that of the tube main body 8 to the flexible tube 5. Such a configuration can address the case where the tubular portion 2c and the bending portion 2b are intended to have different rigidities, for example. The configuration has a larger number of parameters for controlling the characteristics, which enhances the degree of freedom when the characteristics of the flexible tube 5 are optimized.

The flexible tube 5 in the present embodiment can be manufactured inexpensively by using the extruder, the mold having the vacuum mechanism, etc., similarly to the conventional corrugated tube.

### [Second Embodiment]

FIG. 5 shows a configuration, in a planarly developed state, of an outer circumferential surface 5A of a flexible tube 5 in a second embodiment of the present disclosure. In the second embodiment, the same components as those in the first embodiment are attached with the same reference signs and descriptions thereof will be omitted as appropriate. In the second embodiment, description will be made mainly on points different from the first embodiment.

As shown in FIG. 5, a plurality of protruded surfaces 6 each have the same shape, and form a rectangle 6b when the outer circumferential surface 5A of the flexible tube 5 is planarly developed. As is well known, the rectangle 6b has four corners, all of which have right angles, and except for the case where the rectangle 6b is a square, the rectangle 6b has a pair of opposing long sides and a pair of opposing short sides.

The rectangles 6b constituting the plurality of protruded surfaces 6 each include two kinds of rectangles, i.e., a rectangle 6b1 with long sides thereof being arranged in a direction from the upper left side to the lower right side and a rectangle 6b2 with long sides thereof being arranged in a direction from the upper right side to the lower left side.

In addition, the recessed surfaces 7 each include a first inclined part 7b1 (part surrounded by a dotted line in FIG. 5) inclined with respect to the axial direction, along the long side of the rectangle 6b1, and a second inclined part 7b2 (the part surrounded by the one-dot chain line in FIG. 5) inclined with respect to the axial direction, along the long side of the rectangle 6b2.

The first inclined part 7b1 has both ends that abut against the rectangle 6b2. The second inclined part 7b2 has both ends that abut against the rectangle 6b1. Thus, the first inclined parts 7b1 and the second inclined parts 7b2 are formed intermittently, and the length of these in the axial direction is shorter than the length of the flexible tube 5 in the axial direction. The first inclined parts 7b1 and the second inclined parts 7b2 that are formed intermittently have an angle smaller than 360 degrees in the circumferential direction.

The second embodiment provides substantially the same effects as those of the first embodiment.

In addition, in the first embodiment, the spiral-shaped first inclined part 7a1 and the spiral-shaped second inclined part 7a2 are each continuous in the axial direction of the flexible tube 5. In contrast, the first inclined parts 7b1 and the second inclined parts 7b2 in the second embodiment are formed intermittently in the axial direction. With such a configuration, in the flexible tube 5 in the second embodiment, a degree of freedom in controlling the characteristics such as the bendability, the extension/contraction resistance, and the torque resistance can be more enhanced.

Note that, in FIG. 5, the rectangles 6b are each arranged with the respective sides thereof inclined with respect to the axial direction, but are not limited to such an arrangement. The rectangles 6b may each be arranged with the one pair of opposing sides being along the axial direction and the other pair of opposing sides being along the circumferential direction. In addition, the plurality of respective rectangles 6b are not limited to have the same size. The rectangles 6b may have different sizes according to the positions thereof, and the like.

### [Third Embodiment]

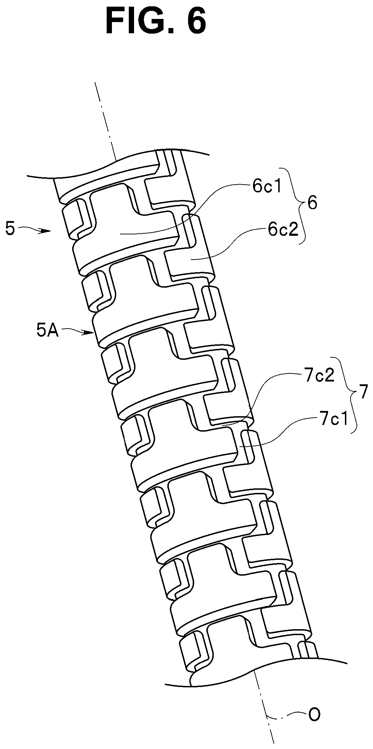

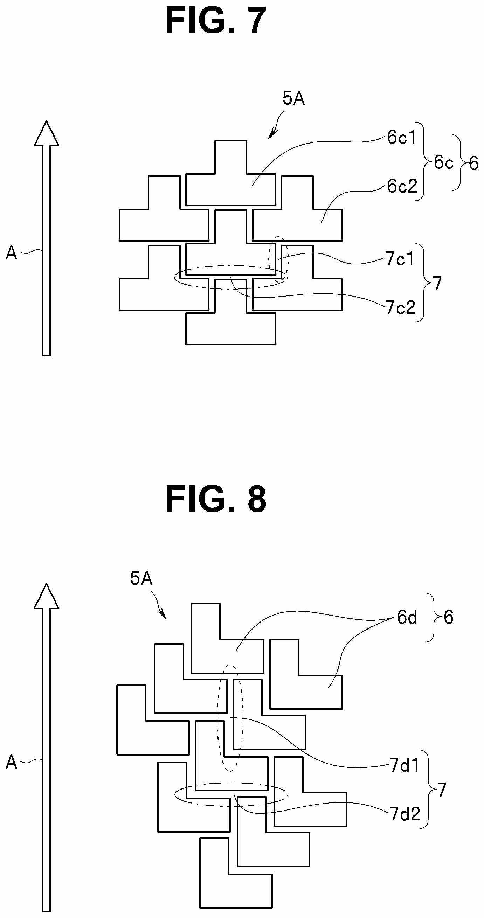

FIG. 6 and FIG. 7 show a third embodiment of the present disclosure. FIG. 6 is a perspective view showing a flexible tube 5 in the third embodiment. FIG. 7 shows a configuration, in a planarly developed state, of an outer circumferential surface 5A of the flexible tube 5 in the third embodiment.

In the third embodiment, the same components as those in the first and second embodiments are attached with the same reference signs and descriptions thereof will be omitted as appropriate. In the third embodiment, description will be made mainly on points different from the first and second embodiments.

As shown in FIG. 6 and FIG. 7, each of a plurality of protruded surfaces 6 forms a T-shape 6c when the outer circumferential surface 5A of the flexible tube 5 is planarly developed. The T-shape 6c is a concave octagon (concave polygon) having two interior angles (specifically, interior angles of 270 degrees) which are reentrant angles (angles larger than 180 degrees and smaller than 360 degrees), and other six interior angles are 90 degrees.

The T-shape 6c is arranged with the long side (the longest side) being along the circumferential direction. In such an arrangement, the eight sides of the T-shape 6c are parallel to either the axial direction or the circumferential direction. Then, recessed surfaces 7 include first parts 7c1 along the axial direction (one first part 7c1 is shown by surrounding it by the dotted line in FIG. 7) and second parts 7c2 along the circumferential direction (one second part 7c2 is shown by surrounding it by the one-dot chain line in FIG. 7).

In other words, the recessed surfaces 7 each include at least one of the first part 7c1 or the second part 7c2. The first parts 7c1 are formed intermittently in the axial direction. Therefore, the length of the first parts 7c1 in the axial direction is shorter than the length of the entire flexible tube 5 in the axial direction. The second parts 7c2 are formed intermittently in the circumferential direction. Therefore, the length of the second parts 7c2 in the circumferential direction is shorter than the length of the outer circumferential surface 5A of the flexible tube 5 in the circumferential direction.

At this time, the depth of the first part 7c1 with respect to the protruded surface 6 may be made different from the depth of the second part 7c2 with respect to the protruded surface 6. In addition, the length of the first part 7c1 may be made different from the length of the second part 7c2. Furthermore, the width of the first part 7c1 may be made different from the width of the second part 7c2.

Among the plurality of T-shapes 6c, the two T-shapes 6c adjacent to each other in the circumferential direction are arranged such that arrangement pitches thereof in the axial direction are shifted by 1/2 pitches from each other. In other words, the plurality of T-shapes 6c include a T-shape 6c1 and a T-shape 6c2, the arrangement pitches thereof in the axial direction are shifted by 1/2 pitches from each other.

The third embodiment provides substantially the same effects as those of the first and second embodiments.

In addition, according to the third embodiment, the first parts 7c1 along the axial direction and the second parts 7c2 along the circumferential direction are provided. With such a configuration, adjusting the depths, the lengths, and the widths of the first parts 7c1 and the second parts 7c2 enables the degree of freedom in controlling the characteristics to be enhanced.

### [Fourth Embodiment]

FIG. 8 shows a configuration, in a planarly developed state, of an outer circumferential surface 5A of a flexible tube 5 in a fourth embodiment of the present disclosure. In the fourth embodiment, the same components as those in the first to third embodiments are attached with the same reference signs and descriptions thereof will be omitted as appropriate. In the fourth embodiment, description will be made mainly on points different from the first to third embodiments.

As shown in FIG. 8, each of a plurality of protruded surfaces 6 forms an L-shape 6d, when the outer circumferential surface 5A of the flexible tube 5 is planarly developed. The L-shape 6d is a concave hexagon (concave polygon) having one interior angle (specifically, interior angle of 270 degrees) which is a reentrant angle, and other five interior angles are 90 degrees.

The L-shape 6d is arranged such that one of the two long sides is along the axial direction and the other is along the circumferential direction. In such an arrangement, the six sides of the L-shape 6d are parallel to either the axial direction or the circumferential direction. Then, recessed surfaces 7 include first parts 7d1 along the axial direction (one first part 7d1 is shown by surrounding it by the dotted line in FIG. 8) and second parts 7d2 along the circumferential direction (one second part 7d2 is shown by surrounding it by the one-dot chain line in FIG. 8).

In other words, the recessed surfaces 7 each include at least one of the first part 7d1 or the second part 7d2. The first parts 7d1 are formed intermittently in the axial direction. Therefore, the length of the first parts 7d1 in the axial direction is shorter than the length of the entire flexible tube 5 in the axial direction. The second parts 7d2 are formed intermittently in the circumferential direction. Therefore, the length of the second parts 7d2 in the circumferential direction is shorter than the length of the outer circumferential surface 5A of the flexible tube 5 in the circumferential direction.

At this time, the depth of the first part 7d1 with respect to the protruded surface 6 may be made different from the depth of the second part 7d2 with respect to the protruded surface 6. In addition, the length of the first part 7d1 may be made different from the length of the second part 7d2. Furthermore, the width of the first part 7d1 may be made different from the width of the second part 7d2.

Note that FIG. 8 shows the L-shapes 6d each including the sides along the axial direction and the sides along the circumferential direction. This configuration does not have a plane symmetry to the plane including the center axis O. Therefore, each of the L-shapes 6d may be rotated, for example, by 45 degrees to be a V-shape, so as to create the plane symmetry to the plane including the center axis O.

The fourth embodiment provides substantially the same effects as those of the first to third embodiments.

In addition, according to the fourth embodiment, the first parts 7d1 along the axial direction and the second parts 7d2 along the circumferential direction are provided. With such a configuration, adjusting the depths, the lengths, and the widths of the first parts 7d1 and the second parts 7d2 enables the degree of freedom in controlling the characteristics to be enhanced.

### [Fifth Embodiment]

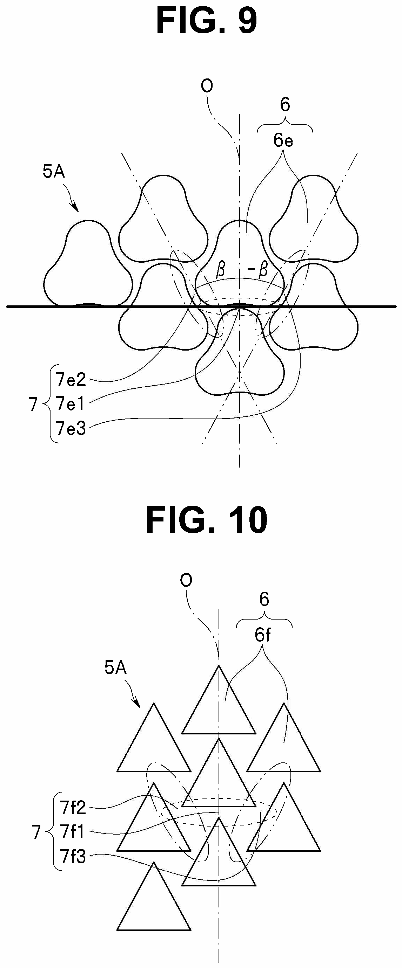

FIG. 9 shows a configuration, in a planarly developed state, of an outer circumferential surface 5A of a flexible tube 5 in a fifth embodiment of the present disclosure. In the fifth embodiment, the same components as those in the first to fourth embodiments are attached with the same reference signs and descriptions thereof will be omitted as appropriate. In the fifth embodiment, description will be made mainly on points different from the first to fourth embodiments.

As shown in FIG. 9, each of a plurality of protruded surfaces 6 forms a rounded polygon when the outer circumferential surface 5A of the flexible tube 5 is planarly developed. The rounded polygon is roughly a shape that is formed by rounding corners of a polygon. In this case, the respective sides of the polygon are not limited to linear lines, and may be a concave arc toward the center of the rounded polygon, for example.

The example shown in FIG. 9 specifically shows rounded triangles 6e (for example, rounded equilateral triangles) each having three sides that form concave arcs. The rounded triangle 6e is arranged with a first side of the three sides being along the circumferential direction. In this arrangement, among the three sides of the rounded triangle 6e, a second side forms an angle β with respect to the axial direction and a third side forms an angle of - β with respect to the axial direction. When the rounded triangle 6e is a rounded equilateral triangle, for example, β is equal to 30 degrees (that is, 45 degrees or less).

Recessed surfaces 7 include first parts 7e1 along the first side (one first part 7e1 is shown by surrounding it by the dotted line in FIG. 9), second parts 7e2 along the second side (one second part 7e2 is shown by surrounding it by the one-dot chain line in FIG. 9), and third parts 7e3 along the third side (one third part 7e3 is shown by surrounding it by the two-dot chain line in FIG. 9).

The first parts 7e1 are formed intermittently in the circumferential direction. Therefore, the length of the first parts 7e1 in the circumferential direction is shorter than the length of the outer circumferential surface 5A of the flexible tube 5 in the circumferential direction. The second parts 7e2 and the third parts 7e3 are each formed intermittently in the axial direction and the circumferential direction. Therefore, the lengths of the second parts 7e2 and the third parts 7e3 in the axial direction are shorter than the length of the entire flexible tube 5 in the axial direction. In addition, angular ranges of the second parts 7e2 and the third parts 7e3 in the circumferential direction are smaller than 360 degrees.

At this time, the depths of the first part 7e1, the second part 7e2, and the third part 7e3 with respect to the protruded surface 6 may be made different from one another. In addition, the lengths of the first part 7e1, the second part 7e2, and the third part 7e3 may be made different from one another. Furthermore, the widths of the first part 7e1, the second part 7e2, and the third part 7e3 may be made different from one another.

The fifth embodiment provides substantially the same effects as those of the first to fourth embodiments.

In addition, according to the fifth embodiment, the first parts 7e1, the second parts 7e2, and the third parts 7e3 that are oriented in the different directions are provided. With such a configuration, adjusting the depths, the lengths, and the widths of the first parts 7e1, the second parts 7e2, and the third parts 7e3 enables the degree of freedom in controlling the characteristics to be enhanced.

### [Sixth Embodiment]

FIG. 10 shows a configuration, in a planarly developed state, of an outer circumferential surface 5A of a flexible tube 5 in a sixth embodiment of the present disclosure. In the sixth embodiment, the same components as those in the first to fifth embodiments are attached with the same reference signs and descriptions thereof will be omitted as appropriate. In the sixth embodiment, description will be made mainly on points different from the first to fifth embodiments.

As shown in FIG. 10, each of a plurality of protruded surfaces 6 forms a polygon, when the outer circumferential surface 5A of the flexible tube 5 is planarly developed. The example shown in FIG. 10 specifically shows triangles 6f (for example, equilateral triangles).

In other words, the flexible tube 5 in the present embodiment has a configuration using the triangles 6f, instead of the rounded triangles 6e in the fifth embodiment shown in FIG. 9.

The triangles 6f each include, for example, a first side along the circumferential direction, and a second side and a third side that intersect the axial direction (and the circumferential direction). When the triangle 6f is an equilateral triangle, for example, the second side and the third side intersect the axial direction at 30 degrees and -30 degrees, respectively.

Recessed surfaces 7 include first parts 7f1 along the first side (one first part 7f1 is shown by surrounding it by the dotted line in FIG. 10), second parts 7f2 along the second side (one second part 7f2 is shown by surrounding it by the one-dot chain line in FIG. 10), and third parts 7f3 along the third side (one third part 7f3 is shown by surrounding it by the two-dot chain line in FIG. 10).

The first parts 7f1 are formed intermittently in the circumferential direction. Therefore, the length of the first parts 7f1 in the circumferential direction is shorter than the length of the outer circumferential surface 5A of the flexible tube 5 in the circumferential direction. The second parts 7f2 and the third parts 7f3 are each formed intermittently in the axial direction and the circumferential direction. Therefore, the lengths of the second parts 7f2 and the third parts 7f3 in the axial direction are shorter than the length of the entire flexible tube 5 in the axial direction. In addition, angular ranges of the second parts 7f2 and the third parts 7f3 in the circumferential direction are smaller than 360 degrees.

At this time, the depths of the first part 7f1, the second part 7f2, and the third part 7f3 with respect to the protruded surface 6 may be made different from one another. In addition, the lengths of the first part 7f1, the second part 7f2, and the third part 7f3 may be made different from one another. Furthermore, the widths of the first part 7f1, the second part 7f2, and the third part 7f3 may be made different from one another.

The sixth embodiment provides substantially the same effects as those of the first to fifth embodiments.

In addition, according to the sixth embodiment, the first parts 7f1, the second parts 7f2, and the third parts 7f3 that are oriented in the different directions are provided. With such a configuration, adjusting the depths, the lengths, and the widths of the first parts 7d1, the second parts 7f2, and the third parts 7f3 enables the degree of freedom in controlling the characteristics to be enhanced.

### [Seventh Embodiment]

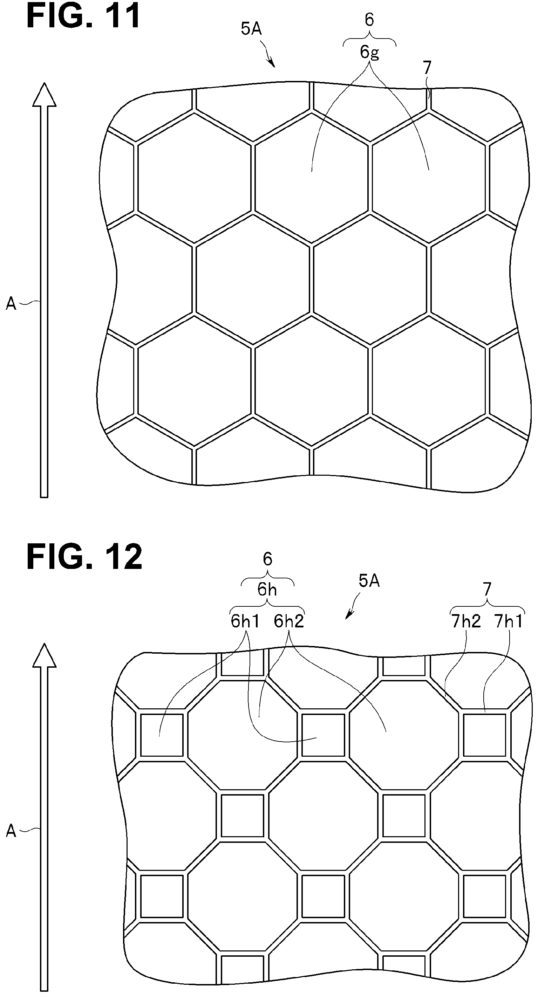

FIG. 11 shows a configuration, in a planarly developed state, of an outer circumferential surface 5A of a flexible tube 5 in a seventh embodiment of the present disclosure. In the seventh embodiment, the same components as those in the first to sixth embodiments are attached with the same reference signs and descriptions thereof will be omitted as appropriate. In the seventh embodiment, description will be made mainly on points different from the first to sixth embodiments.

As shown in FIG. 11, each of a plurality of protruded surfaces 6 forms a polygon, when the outer circumferential surface 5A of the flexible tube 5 is planarly developed. The example shown in FIG. 10 specifically shows hexagons 6g (for example, regular hexagons).

The hexagons 6g shown in FIG. 11 are subjected to tiling such that, among three pairs of opposing sides of each of the hexagons 6g, a first pair of sides is parallel to the axial direction. When the hexagon 6g is a regular hexagon, for example, a second pair of sides and a third pair of sides intersect the axial direction at 60 degrees and -60 degrees, respectively.

Recessed surfaces 7 are provided between the hexagons 6g. At this time, the depths with respect to the protruded surfaces 6, the widths, and the like of the recessed surfaces 7 along the first pair of sides, the recessed surfaces 7 along the second pair of sides, and the recessed surfaces 7 along the third pair of sides may be made different from one another. In addition, the lengths of the respective recessed surfaces 7 may be made different from one another by employing the hexagons 6g other than the regular hexagons.

The seventh embodiment provides substantially the same effects as those of the first to sixth embodiments.

According to the seventh embodiment, adjusting the depths, the lengths, and the widths of the recessed surfaces 7 according to the positions where the recessed surfaces 7 are provided enables the degree of freedom in controlling the characteristics to be enhanced.

### [Eighth Embodiment]

FIG. 12 shows a configuration, in a planarly developed state, of an outer circumferential surface 5A of a flexible tube 5 in an eighth embodiment of the present disclosure. In the eighth embodiment, the same components as those in the first to seventh embodiments are attached with the same reference signs and descriptions thereof will be omitted as appropriate. In the eighth embodiment, description will be made mainly on points different from the first to seventh embodiments.

In each of the first to seventh embodiments, the plurality of protruded surfaces 6 respectively have the same shape. In contrast, in the present embodiment, a configuration is employed in which tiling with protruded surfaces 6 is performed by combining the protruded surfaces 6 of two different types of shapes.

When the outer circumferential surface 5A of the flexible tube 5 is planarly developed, the plurality of protruded surfaces 6 include, for example, two types of polygons 6h, i.e., a quadrangle 6h1 (for example, square) and an octagon 6h2 (for example, regular octagon)

When the quadrangle 6h1 is a square and the octagon 6h2 is a regular octagon, the quadrangle 6h1 has four sides whose lengths are equal, the octagon 6h2 has eight sides whose lengths are equal, and the length of one side of the quadrangle 6h1 is equal to the length of one side of the octagon 6h2.

In the example shown in FIG. 12, the plurality of protruded surfaces 6 are arranged such that, of two pairs of sides of each of the quadrangles 6h1, a first pair of sides is in the axial direction and a second pair of sides is in the circumferential direction.

Recessed surfaces 7 include a first part 7h1 disposed between the quadrangle 6h1 and the octagon 6h2, and a second part 7h2 disposed between the octagons 6h2 adjacent to each other.

The depths, the lengths, and the widths of the first part 7h1 and the second part 7h2 may be made different according to the positions, directions (axial direction, circumferential direction, diagonal direction, etc.) and the like where the first part 7h1 and the second part 7h2 are disposed on the flexible tube 5.

The eighth embodiment provides substantially the same effects as those of the first to seventh embodiments.

According to the eighth embodiment, adjusting the depths, the lengths, and the widths of the recessed surfaces 7 according to the positions and directions where the recessed surfaces 7 are provided enables the degree of freedom in controlling the characteristics to be enhanced.

Note that FIG. 12 shows the example in which the plurality of protruded surfaces 6 include two types of shapes, i.e., the quadrangle 6h1 and the octagon 6h2. However, another types of shapes may be combined. Furthermore, the shapes of the plurality of protruded surfaces 6 are not limited to two types, and may be three types or more.

### [Ninth Embodiment]

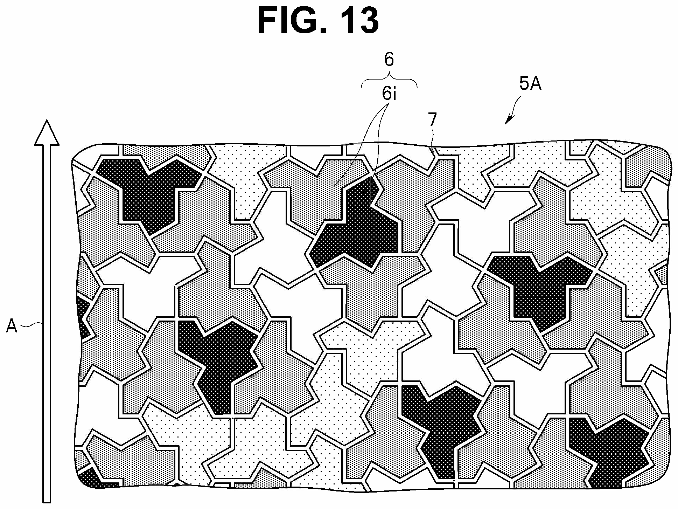

FIG. 13 shows a configuration, in a planarly developed state, of an outer circumferential surface 5A of a flexible tube 5 in a ninth embodiment of the present disclosure. In the ninth embodiment, the same components as those in the first to eighth embodiments are attached with the same reference signs and descriptions thereof will be omitted as appropriate. In the ninth embodiment, description will be made mainly on points different from the first to eighth embodiments.

In the first to seventh embodiments, the protruded surfaces 6 of the one type of shape are periodically aligned, and in the eighth embodiment, the protruded surfaces 6 of the two types of shapes are periodically aligned. In contrast, in the present embodiment, a configuration is employed in which tiling with protruded surfaces 6 is performed by arranging the protruded surfaces of one type of shape aperiodically.

The plurality of protruded surfaces 6 are arranged aperiodically along the outer circumferential surface 5A. The protruded surfaces 6 shown in FIG. 13 are each a concave tridecagon (concave polygon) 6i having four interior angles which are reentrant angles. Note that, in FIG. 13, although different hatching patterns are used for easy discrimination among the plurality of concave tridecagons 6i, the plurality of concave tridecagons 6i have the same shape.

Specifically, the concave tridecagon 6i is also called as "Smith's hat", and recited in the website shown below, for example.

A chiral aperiodic monotile [David Smith1, Joseph Samuel Myers2, Craig S. Kaplan3, and Chaim Goodman-Strauss4] arXiv: 2305.17743v1 [math.CO] 28 May 2023 [retrieved on 2023-09-07] Retrieved from the Internet: (URL: https://arxiv.org/pdf/2305.17743.pdf)

In addition, recessed surfaces 7 are each arranged between the adjacent concave tridecagons 6i.

Note that the concave tridecagons 6i shown in FIG. 13 having the same shape can be arranged aperiodically. However, another example is also known in which even if the shapes are the same, both front and back sides can be combined and arranged aperiodically. Such an example is recited in the website shown below, for example. Such a configuration combining the front and back sides may be applied to the flexible tube 5.

An aperiodic monotile [David Smith1, Joseph Samuel Myers*2, Craig S. Kaplan3, and Chaim Goodman-Strauss4] arXiv:2303.10798v2 [math. CO] 29 May 2023 [retrieved on 2023-09-07] Retrieved from the Internet: (URL: https://arxiv.org/pdf/2303.10798.pdf)

Furthermore, an example called penrose tiling is also known in which figures are arranged aperiodically by combining two types of shapes. Such a configuration combining a plurality of types of shapes may be applied to the flexible tube 5.

The ninth embodiment provides substantially the same effects as those of the first to eighth embodiments.

According to the ninth embodiment, it is possible to obtain a characteristic which is substantially even from a broad perspective but uneven from a local perspective.

### [Tenth Embodiment]

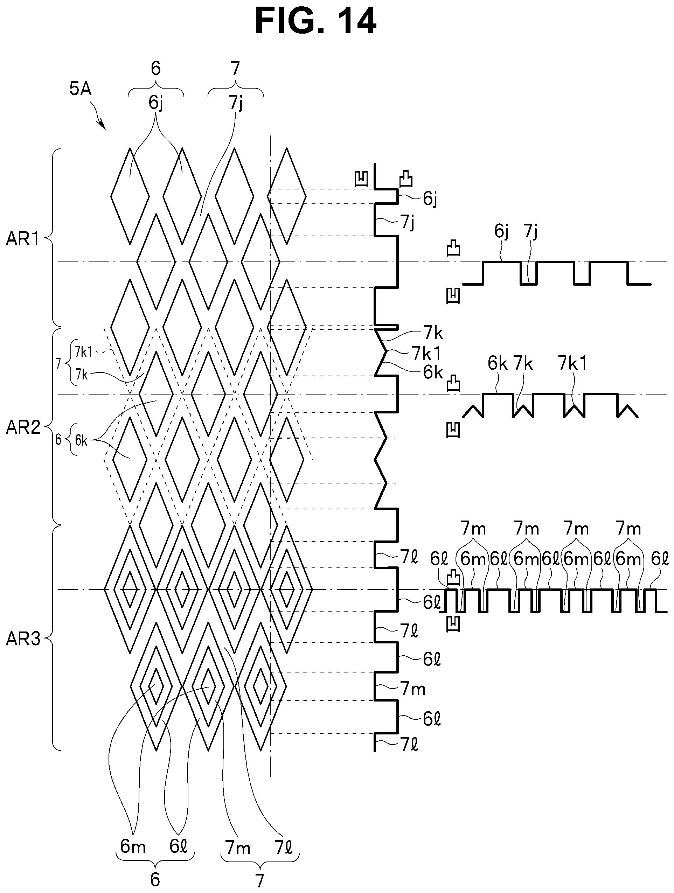

FIG. 14 shows a configuration, in a planarly developed state, of an outer circumferential surface 5A of a flexible tube 5 in a tenth embodiment of the present disclosure. In the tenth embodiment, the same components as those in the first to ninth embodiments are attached with the same reference signs and descriptions thereof will be omitted as appropriate. In the tenth embodiment, description will be made mainly on points different from the first to ninth embodiments.

In the first to ninth embodiments, the two-dimensional shape of the protruded surfaces 6 (further, the recessed surfaces 7) is constant, in principle, regardless of the positions thereof in the axial direction. In contrast, in the present embodiment, two-dimensional shape of protruded surfaces 6 (further, recessed surfaces 7) is changed along the axial direction.

As shown in FIG. 14, the flexible tube 5 includes a first area AR1, a second area AR2, and a third area AR3 along the axial direction.

Note that FIG. 14 shows also a chart showing surface shapes in the cases where the outer circumferential surface 5A of the flexible tube 5, which has been planarly developed, is cut respectively along four one-dot chain lines (a one-dot chain line in the axial direction, a one-dot chain line in the circumferential direction in the first area AR1, a one-dot chain line in the circumferential direction in the second area AR2, and a one-dot chain line in the circumferential direction in the third area AR3).

The first area AR1 is an area where the bendability is relatively low (that is, rigid), for example. Specifically, each of the plurality of protruded surfaces 6 aligned in the first area AR1 forms a rhombus 6j, when the outer circumferential surface 5A of the flexible tube 5 is planarly developed. The recessed surfaces 7j are each provided between the adjacent rhombuses 6j.

The second area AR2 is an area where the bendability is relatively medium (that is, middle rigidity), for example. Specifically, each of a plurality of protruded surfaces 6 aligned in the second area AR2 forms a rhombus 6k, when the outer circumferential surface 5A of the flexible tube 5 is planarly developed. An area of one rhombus 6k is smaller than an area of one rhombus 6j, for example.

Recessed surfaces 7k are each provided between the adjacent rhombuses 6k. The recessed surface 7k includes, at the center thereof, a chevron 7k1. A height of the chevron 7k1 is lower than a height of the rhombus 6k. In other words, also the chevron 7k1 is a part of the recessed surface 7k when viewed from the rhombus 6k which is the protruded surface 6. The chevron 7k1 is provided, which enables the recessed surface 7k to be bent easily, and increases the extension/contraction property of the recessed surface 7k in the direction orthogonal to the chevron 7k1.

A ratio of the area of the protruded surfaces 6 to the area of the recessed surfaces 7 for each of the areas is larger in the first area AR1 than in the second area AR2, for example.

The third area AR3 is an area where the bendability is relatively high (that is, soft), for example. Comparison among the bendabilities in the respective areas AR1 to AR3 provides the following. In the axial direction and the circumferential direction, the number of protrusions and recesses per unit length is larger in the second area AR2 than in the first area AR1, and larger in the third area AR3 than in the second area AR2. With such a configuration, the bendability is higher in the second area AR2 than in the first area AR1, and higher in the third area AR3 than in the second area AR2.

The plurality of protruded surfaces 6 aligned in the third area AR3 each include a first protruded surface 6l and an inner rhombus 6m, when the outer circumferential surface 5A of the flexible tube 5 is planarly developed.

The first protruded surfaces 6l are protruded surfaces formed by connecting a plurality of ring-shaped rhombuses (hereinafter, referred to as outer rhombuses) in the circumferential direction. It is understood that the plurality of outer rhombuses are connected in the circumferential direction from the chart showing the surface shape when the outer circumferential surface 5A is cut along the one-dot chain line in the circumferential direction in the third area AR3. However, the plurality of outer rhombuses may be isolated from one another, instead of connecting them in the circumferential direction.

The inner rhombuses 6m are protruded surfaces each arranged in the inside of each of the plurality of outer rhombuses of the first protruded surfaces 61, spaced apart from each of the plurality of outer rhombuses without being connected thereto. Thus, inside a given protruded surface 6, another protruded surface 6 may be provided.

Recessed surfaces 7l are each provided between the first protruded surfaces 6l adjacent to each other in the axial direction. In addition, recessed surfaces 7m are each provided between the outer rhombus of the first protruded surface 6l and the inner rhombus 6m. Thus, the first protruded surface 6l and the inner rhombus 6m are each surrounded by the recessed surface 7 (recessed surface 7l or the recessed surface 7m) and isolated from another protruded surface 6.

Accordingly, each of the plurality of protruded surfaces 6 has a first shape in a first range (for example, any one area of the first area AR1, the second area AR2, and the third area AR3) along the axial direction. In addition, each of the plurality of protruded surfaces 6 has a second shape different from the first shape in a second range (for example, another one area of the first area AR1, the second area AR2, and the third area AR3) along the axial direction, which is different from the first range.

Note that, in the above-described example, the shape patterns of the protruded surfaces 6 and recessed surfaces 7 are changed for each of the areas, but are not limited to such an example. For example, morphing may be used to configure the shape patterns of the protruded surfaces 6 and recessed surfaces 7 change smoothly along the axial direction.

The tenth embodiment provides substantially the same effects as those of the first to ninth embodiments.

According to the tenth embodiment, the shape patterns of the protruded surfaces 6 and the recessed surfaces 7 are changed in the axial direction. With such a configuration, depending on how to change the shape patterns, the characteristics such as the bendability, the extension/contraction resistance, and the torque resistance in the axial direction can be controlled at a higher degree of freedom in the axial direction. Such a configuration enables the one flexible tube 5 to have both the area suitable for the tubular portion 2c and the area suitable for the bending portion 2b, for example.

### [Eleventh Embodiment]

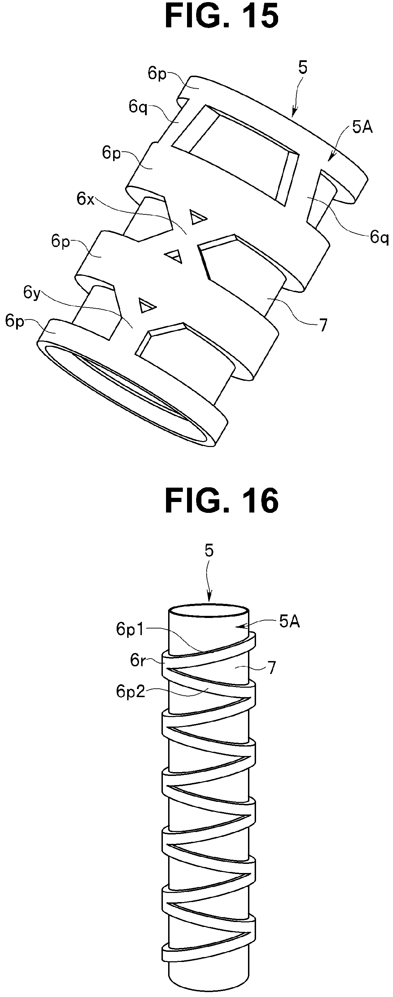

FIG. 15 is a perspective view showing a flexible tube 5 in an eleventh embodiment of the present disclosure. In the eleventh embodiment, the same components as those in the first to tenth embodiments are attached with the same reference signs and descriptions thereof will be omitted as appropriate. In the eleventh embodiment, description will be made mainly on points different from the first to tenth embodiments.

The flexible tube 5 in each of the first to tenth embodiments includes the plurality of protruded surfaces 6 each surrounded by the recessed surfaces 7 to be isolated from one another. In contrast, the flexible tube 5 in the present embodiment is formed by connecting a plurality of protruded surfaces 6 each forming a ring shape in the circumferential direction to one another in the axial direction.

The flexible tube 5 can be applied to the insertion portion 2 including the bending portion 2b and the tubular portion 2c. If a corrugated tube having a bellows structure is used for the tubular portion 2c, for example, it can contribute to an improvement of the procedure (excellent bending performance, reduction of fatigue of an operator) due to a light weight and an excellent bendability of such a corrugated tube.

However, the corrugated tube extends and contracts in the axial direction. Therefore, if the corrugated tube is used for the insertion portion 2, when a push/pull operation is performed in a procedure using a lower gastrointestinal endoscope, for example, a difference occurs between a sense of insertion of an operator and an actual insertion length, which results in a degradation of the operability in the procedure. In view of the above, a configuration of the flexible tube 5 whose extension/contraction resistance is improved than that of the corrugated tube will be described with reference to FIG. 15.

The flexible tube 5 shown in FIG. 15 includes a plurality of protruded surfaces 6p each forming a ring shape in the circumferential direction. Recessed surfaces 7 are each arranged between the protruded surfaces 6p adjacent to each other. Note that filling structure parts 9 may be formed respectively in the recessed surfaces 7 by filling the recessed surfaces 7 with a filling material, similarly to the above-described embodiments.

The two adjacent protruded surfaces 6p are connected with a connecting protruded surface. The two adjacent protruded surfaces 6p are relatively hard to extend and contract at the part where the connecting protruded surface is provided, and relatively easy to extend and contract at the part where the connecting protruded surface is not provided.

For example, the two adjacent protruded surfaces 6p are connected to each other by using a plurality of connecting protruded surfaces 6q that are inclined with respect to the axial direction (that is, not parallel to the axial direction). For example, the two connecting protruded surfaces 6q are inclined at respective angles symmetrical to the axial direction.

In a first example shown in FIG. 15, the plurality of connecting protruded surfaces 6q are arranged apart from one another in the circumferential direction. The connecting protruded surfaces 6q are connected to each of the two protruded surfaces 6p at a specific angle.

Note that when only the two connecting protruded surfaces 6q are arranged at the positions shown in FIG. 15, the two connecting protruded surfaces 6q are not arranged at the 180-degree opposite positions in the circumferential direction. Arranging the connecting protruded surfaces 6q at uneven and deviated positions around the center axis O enables control of a direction in which bending is easy and a direction in which bending is hard.

In addition, in a second example shown in FIG. 15, the two adjacent protruded surfaces 6p are connected to each other by using a connecting protruded surface 6x formed in an X-shape, for example. The connecting protruded surface 6x is configured such that a plurality of (two in the example shown in the drawing) protruded surfaces 6 are arranged so as to intersect each other. Note that the position where the plurality of protruded surfaces 6 intersect each other is not limited to the center position of the connecting protruded surface 6x (center position of the connecting protruded surface 6x in the axial direction). Also in this case, the connecting protruded surface 6x is connected to each of the two protruded surfaces 6p at a specific angle.

Furthermore, in a third example shown in FIG. 15, the two adjacent protruded surfaces 6p are connected to each other by using a connecting protruded surface 6y formed in a Y-shape, for example. The connecting protruded surface 6y is formed such that a plurality of protruded surfaces 6 are merged at a halfway point, to form one protruded surface. Note that the position where the plurality of protruded surfaces 6 are merged is not limited to the center position of the connecting protruded surface 6y. In this case, the connecting protruded surface 6y is configured such that two branched parts are connected to one of the protruded surfaces 6p at a specific angle. In addition, the merged part of the connecting protruded surface 6y is connected, for example, orthogonally to the other of the protruded surfaces 6p.

Note that FIG. 15 shows the example in which the three types of connecting protruded surfaces 6q, 6x, and 6y are provided. However, needless to say, only any one or two types of the connecting protruded surfaces may be provided.

According to the tenth embodiment, the connecting protruded surfaces 6q, 6x, and 6y are provided, which can suppress the extension/contraction characteristic of the corrugated tube in the axial direction. Such a configuration can provide the flexible tube 5 which is suitable for the bending portion 2b and the tubular portion 2c that require the extension/contraction resistance.

In addition, the connecting protruded surfaces 6q, 6x, and 6y each have the part inclined with respect to the axial direction, which can provide the extension/contraction resistance and the torque resistance when a rotation torque around the center axis O is applied to the flexible tube 5. At this time, adjusting the inclined angles enables adjustment of the balance between the extension/contraction resistance and the torque resistance of the flexible tube 5.

Furthermore, adjusting the positions of the connecting protruded surfaces 6q, 6x, and 6y in the circumferential direction enables control of the direction in which the flexible tube 5 is easy to bend and the direction in which the flexible tube 5 is hard to bend. On the other hand, equalizing the positions of the protruded surfaces to be connected in the circumferential direction can equalize the direction in which the flexible tube 5 is easy to bend.

In addition, the connecting protruded surfaces 6q, 6x, and 6y, the positions of which in the axial direction are different from one another, are arranged at different positions in the circumferential direction, which can equalize the easiness of the extension/contraction of the flexible tube.

Furthermore, since the connecting protruded surface 6x formed in the X-shape and the connecting protruded surface 6y formed in the Y-shape each have the branched part, when a stress is applied to the flexible tube 5, the stress acts separately thereon. As a result, the stress that acts on the flexible tube 5 is relieved, which can suppress the extension and contraction of the flexible tube 5.

### [Twelfth Embodiment]

FIG. 16 is a perspective view showing a flexible tube 5 in a twelfth embodiment of the present disclosure. In the twelfth embodiment, the same components as those in the first to eleventh embodiments are attached with the same reference signs and descriptions thereof will be omitted as appropriate. In the twelfth embodiment, description will be made mainly on points different from the first to eleventh embodiments.

The flexible tube 5 in the present embodiment is formed by arranging a plurality of protruded surfaces 6, each of which forms a ring shape in the circumferential direction, so as to be inclined at angles alternately different with respect to the circumferential direction and by connecting the plurality of protruded surfaces 6 at the parts where they are proximity to each other.

The flexible tube 5 includes ring-shaped first protruded surfaces 6p1 inclined at a first angle with respect to the circumferential direction and ring-shaped second protruded surfaces 6p2 inclined at a second angle with respect to the circumferential direction. Each of the first protruded surfaces 6p1 and each of the second protruded surfaces 6p2 are connected to each other by a connecting portion 6r. The connecting portion 6r is a protruded surface on which each of the first protruded surface 6p1 and each of the second protruded surface 6p2 intersect each other.

In other words, when focusing on a given first protruded surface 6p1, the given first protruded surface 6p1 is connected to the second protruded surface 6p2 which is on one side with respect to the axial direction by a first connecting portion 6r, and connected to the second protruded surface 6p2 which is on the other side with respect to the axial direction by a second connecting portion 6r. The first connecting portion 6r and the second connecting portion 6r are located, for example, at opposite positions in the circumferential direction, that is, at the positions different from each other by 180 degrees in the circumferential direction.

For example, if the angle in the circumferential direction at which the first connecting portion 6r is provided is supposed to be 0 degrees and the angle in the circumferential direction at which the second connecting portion 6r is provided is supposed to be 180 degrees, no connecting portion 6r is present at a position between 0 to 180 degrees, and a position between 180 to 0 degrees.

Note that, although the example in which the connecting portions 6r are provided at the positions different from each other by 180 degrees in the circumferential direction is shown here, adjusting the angles in the circumferential direction at which the connecting portions 6r are provided enables control of a direction in which bending is easy and a direction in which bending is hard.

Thus, the first protruded surface 6p1 is connected to the two second protruded surfaces 6p2 that are adjacent in the axial direction at one location each, i.e., at two locations in total. In addition, the second protruded surface 6p2 is connected to the two first protruded surfaces 6p1 that are adjacent in the axial direction at one location each, i.e., at two locations in total.

Recessed surfaces 7 are each provided between each of the first protruded surfaces 6p1 and each of the second protruded surfaces 6p2, except for the connecting portions 6r. Note that filling structure parts 9 may be provided respectively in the recessed surfaces 7 by filling the recessed surfaces 7 with a filling material, similarly to the above-described embodiments.

The twelfth embodiment provides substantially the same effects as those of the above-described eleventh embodiment. In addition, adjusting the inclined angles of the first protruded surfaces 6p1 and the second protruded surfaces 6p2 enables the adjustment of the balance between the extension/contraction resistance and the torque resistance of the flexible tube 5.

### [Thirteenth Embodiment]

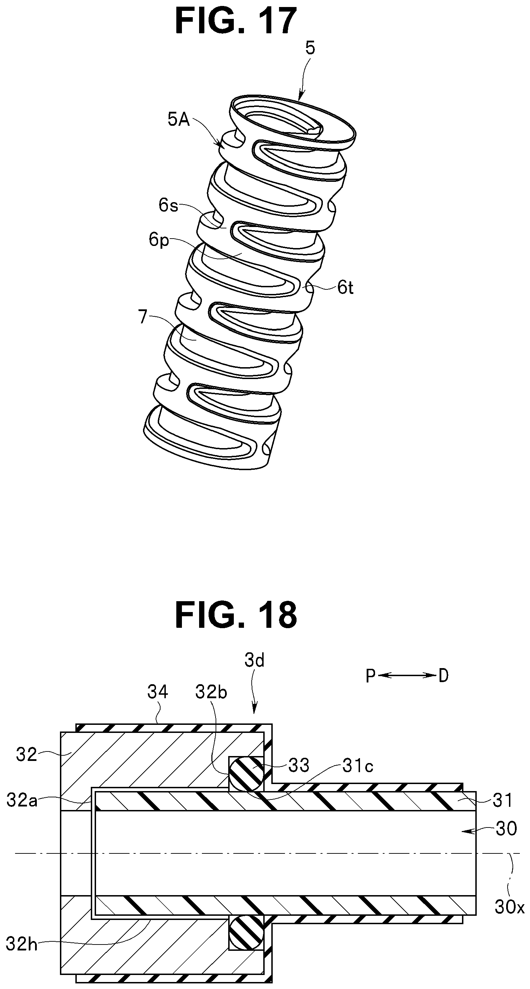

FIG. 17 is a perspective view showing a flexible tube 5 in a thirteenth embodiment of the present disclosure. In the thirteenth embodiment, the same components as those in the first to twelfth embodiments are attached with the same reference signs and descriptions thereof will be omitted as appropriate. In the thirteenth embodiment, description will be made mainly on points different from the first to twelfth embodiments.

The flexible tube 5 in the present embodiment is formed by connecting two protruded surfaces 6, each of which forms a ring shape in the circumferential direction, at a plurality of locations in the circumferential direction.

The flexible tube 5 includes the plurality of protruded surfaces 6p, each of which forms the ring shape in the circumferential direction.

When focusing on a given protruded surface 6p, the given protruded surface 6p is connected to the protruded surface 6p which is on one side with respect to the axial direction at a first connecting portion 6s, and connected to the protruded surface 6p which is on the other side with respect to the axial direction at a second connecting portion 6t. Both the first connecting portion 6s and the second connecting portion 6t are the protruded surfaces 6.

The first connecting portions 6s are provided respectively at two locations different from each other by 180 degrees in the circumferential direction, for example. The second connecting portions 6t are provided respectively at two locations different from each other by 180 degrees in the circumferential direction, for example. The first connecting portions 6s and the second connecting portions 6t differ in the angle around the center axis O by 90 degrees, for example.

With such a configuration, a given protruded surface 6p is connected to the protruded surface 6p which is on the one side with respect to the axial direction by the first connecting portions 6s at the two locations and connected to the protruded surface 6p which is on the other side with respect to the axial direction by the second connecting portions 6t at the two locations. Note that the first connecting portions 6s and the second connecting portions 6t may be provided so as to be inclined with respect to the axial direction or parallel to the axial direction.

Here, a given protruded surface 6p may have a wave-shaped inclination along the circumferential direction such that the given protruded surface 6p is in proximity to the protruded surface 6p on one side at the first connecting portions 6s at the two locations and is in proximity to the protruded surface 6p on the other side at the second connecting portions 6t at the two locations.

Recessed surfaces 7 are each provided between the plurality of protruded surfaces 6p, except for the connecting portions 6s and 6t. Note that filling structure parts 9 may be provided respectively in the recessed surfaces 7 by filling the recessed surfaces 7 with a filling material, similarly to the above-described embodiments.

The thirteenth embodiment provides substantially the same effects as those of the above-described twelfth embodiment.

Note that FIG. 16 of the twelfth embodiment shows the example in which the given protruded surface 6p is connected to the other protruded surfaces 6p at each of the upper one location and the lower one location (two locations in total). In addition, FIG. 17 of the thirteenth embodiment shows the example in which the given protruded surface 6p is connected to the other protruded surfaces 6p at each of the upper two locations and the lower two locations (four locations in total). The embodiments are not limited to these examples, and a configuration may be employed in which a given protruded surface 6p is connected to other protruded surfaces 6p at three locations or more in total.

### [Related Technology]

FIG. 18 to FIG. 26 show a related technology that relates to the endoscope 1.

In the related technology, the same components as those in the above-described embodiments are attached with the same reference signs and descriptions thereof will be omitted as appropriate. In the related technology, description will be made mainly on points different from the respective embodiments.

In the treatment instrument insertion port 3d, for example, of the endoscope 1, the tube constituting the treatment instrument channel inserted into the endoscope 1 is connected to a ferrule (cylinder) provided in the treatment instrument insertion port 3d. Such a connecting part of the tube requires resistances such as a connection strength, a water-tightness, and a pressure resistance. An attempt to meet standards for all of these resistances will result in a complicated structure and increased costs.

For example, there is a case where a bending pipe for relaying the tube and the ferrule is required for ensuring the resistances, and such a case will result in a complicated structure.

When a tapered pipe is used for connecting the tube, fastening with nuts is performed, which results in an increase in assembly man-hours due to the increased number of components, and the difficulty in the fastening work is high. In addition, a torque management and an anti-loosening measure for the nuts are required at the time of fastening the nuts.

Furthermore, when the tube and the ferrule are fixed to each other by adhering, it is difficult to apply an adhesive to the entire circumference of the tube, which may cause adhesion application unevenness. The resistance is high if there is no adhesion application unevenness, but the resistance is lowered if there is an adhesion application unevenness. Therefore, the resistance varies depending on the work, which may be a cause of a variation in the quality. In addition, when an adhesive is used, an adhesive curing time is required, which lengthens the manufacturing time. Furthermore, the adhered part may possibly deteriorate after the adhesion.

Whereas, for single-use endoscopes, a simplified structure and a reduced cost are required. To this end, there are demands for a reduced number of components, a depreciation of a manufacturing facility in a short time, and a high yield rate. A configuration that can cope with such demands will be described with reference to FIG. 18 to FIG. 26.

FIG. 18 is a cross-sectional view showing a first connecting structure of a tube 31 and a rigid member 32 in the related technology. Note that, description will be made below by taking a case where the rigid member 32 is a ferrule provided in the treatment instrument insertion port 3d, as an example. In this case, in FIG. 18 to FIG. 26, the rigid member 32 side (left side in the drawings) is the proximal end side (proximal end direction P side) of the entire endoscope 1, and the tube 31 side (right side in the drawings) is the distal end side (distal end direction D side) of the entire endoscope 1.

The tube 31 is used, for example, for the channels (the treatment instrument channel, the gas/liquid feeding channel, the suction channel, etc.) in the endoscope 1. The rigid member 32 is used, for example, as a ferrule of a channel opening of the endoscope 1. However, the intended uses are not limited to the above-described ones, and the tube 31 and the rigid member 32 may be used for other parts in the endoscope 1, or for a medical device other than the endoscope.

For example, the tube 31 is formed of a resin, for example. The rigid member 32 is formed of a material that is more rigid than that of the tube 31.

The rigid member 32 includes a through hole 32h. The through hole 32h of the rigid member 32 constitutes a part of a path 30 such as the above-described channels. A center axis 30x is an axis of a center of the path 30.

The tube 31 is arranged such that the proximal end side of the tube 31 is disposed in the through hole 32h. An inner part of the tube 31 constitutes another part of the path 30.

The rigid member 32 includes, in the through hole 32h thereof, a receiving surface 32a configured to receive the tube 31 that is inserted into the rigid member 32. The through hole 32h includes, in the opening portion on the distal end thereof, an enlarged-diameter portion 32b. In the enlarged-diameter portion 32b, an O-ring 33 (first O-ring) externally fitted to the tube 31 is arranged. The O-ring 33 is in contact with the rigid member 32 and an outer circumferential surface 31c of the tube 31.

A heat-shrinkable tube 34 is arranged on the outer circumferential sides of the tube 31, the rigid member 32, and the O-ring 33. The heat-shrinkable tube 34 is in contact with the rigid member 32 and the tube 31, and is shrunk in a state of covering the rigid member 32 and the tube 31.

A work for connecting the tube 31 to the rigid member 32 is performed with steps (1) to (5) shown below, for example.
(1) The O-ring 33 is assembled to the outer circumferential surface 31c of the tube 31.
(2) The heat-shrinkable tube 34 is inserted into the tube 31.
(3) The tube 31 is inserted until the proximal end of the tube 31 reaches the receiving surface 32a of the rigid member 32.
(4) The O-ring 33 is abutted to the enlarged-diameter portion 32b of the rigid member 32.
(5) In the state where the heat-shrinkable tube 34 covers the rigid member 32, the O-ring 33, and the proximal end side of the tube 31, heat is applied to the heat-shrinkable tube 34 to cause the heat-shrinkable tube 34 to shrink.

Note that if a tube with an adhesive being applied, in advance, to an inner circumferential surface thereof is used as the heat-shrinkable tube 34, the heat-shrinkable tube 34 can be adhered to an object against which the inner circumferential surface of the heat-shrinkable tube 34 abuts, without the need for a work for applying an adhesive.

According to the first connecting structure, an adhesive property of the heat-shrinkable tube 34 enables the connection strength of the tube 31 and the rigid member 32 to be ensured. In addition, the shrinking of the heat-shrinkable tube 34 causes the O-ring 33 to be compressed toward the radially inward thereof. As a result, a gap is sealed, to thereby enable the water-tightness to be ensured. Furthermore, since even the heat-shrinkable tube 34 itself can keep the water-tightness, combining the heat-shrinkable tube 34 and the O-ring 33 enables the firmer water-tightness to be ensured.

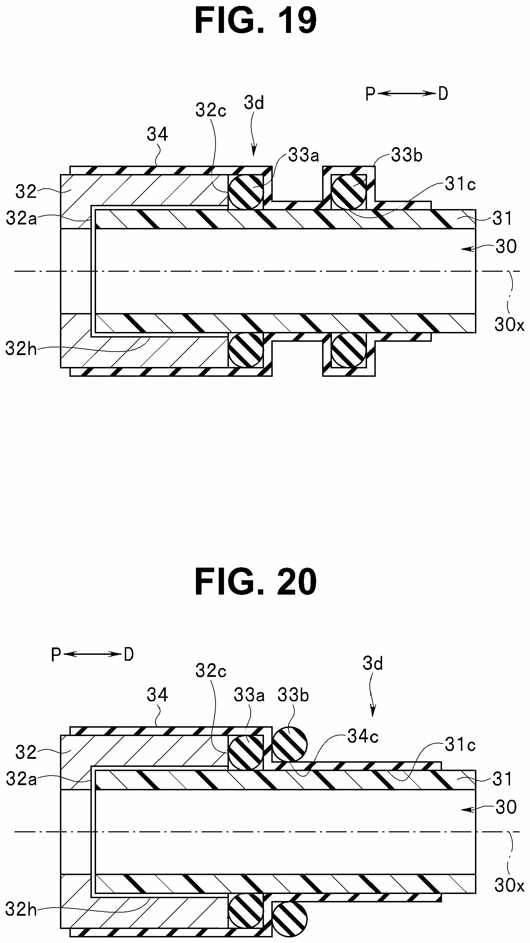

FIG. 19 is a cross-sectional view showing a second connecting structure of the tube 31 and the rigid member 32 in the related technology. In the second connecting structure, the same components as those in the first connecting structure are attached with the same reference signs and descriptions thereof will be omitted as appropriate. In the second connecting structure, description will be made mainly on points different from the first connecting structure.

The rigid member 32 includes the receiving surface 32a configured to receive the tube 31. A first O-ring 33a and a second O-ring 33b are assembled to the outer circumferential surface 31c of the tube 31. Therefore, the first O-ring 33a and the second O-ring 33b contact the outer circumferential surface 31c of the tube 31.

The tube 31 is inserted until the proximal end thereof reaches the receiving surface 32a of the rigid member 32, to cause the first O-ring 33a to abut to a distal end surface 32c of the rigid member 32. The second O-ring 33b is arranged at a position which is more distal end side than the first O-ring 33a and which is a little away from the first O-ring 33a.

In the state where the heat-shrinkable tube 34 covers the rigid member 32, the first O-ring 33a, the second O-ring 33b, and the proximal end side of the tube 31, heat is applied to the heat-shrinkable tube 34 to cause the heat-shrinkable tube 34 to shrink.

The heat-shrinkable tube 34 is in contact with the second O-ring 33b and is shrunk in the state of covering the second O-ring 33b, to compress the second O-ring 33b in a direction toward the center axis 30x of the path 30.

The second connecting structure provides substantially the same effects as those of the first connecting structure.

In addition, according to the second connecting structure, using the plurality of O-rings enables the higher water-tightness and the higher connection strength to be ensured.

FIG. 20 is a cross-sectional view showing a third connecting structure of the tube 31 and the rigid member 32 in the related technology. In the third connecting structure, the same components as those in the first and second connecting structures are attached with the same reference signs and descriptions thereof will be omitted as appropriate. In the third connecting structure, description will be made mainly on points different from the first and second connecting structures.

The rigid member 32 includes the receiving surface 32a configured to receive the tube 31. The first O-ring 33a and the second O-ring 33b are assembled to the outer circumferential surface 31c of the tube 31. At this time, the second O-ring 33b is arranged at a position away from the first O-ring 33a.

The tube 31 is inserted until the proximal end thereof reaches the receiving surface 32a of the rigid member 32, to cause the first O-ring 33a to abut to the distal end surface 32c of the rigid member 32.

In the state where the heat-shrinkable tube 34 covers the rigid member 32, the first O-ring 33a, and the tube 31, and does not cover the second O-ring 33b, heat is applied to the heat-shrinkable tube 34 to cause the heat-shrinkable tube 34 to shrink.

After that, the second O-ring 33b is moved to a position where the second O-ring 33b faces the first O-ring 33a across the heat-shrinkable tube 34. Then, the second O-ring 33b is in contact with an outer circumferential surface 34c of the heat-shrinkable tube 34 that covers the tube 31.

The third connecting structure provides substantially the same effects as those of the first and second connecting structures.

In addition, according to the third connecting structure, the second O-ring 33b presses the heat-shrinkable tube 34 from the outer circumferential side of the heat-shrinkable tube 34, which can ensure high water-tightness.

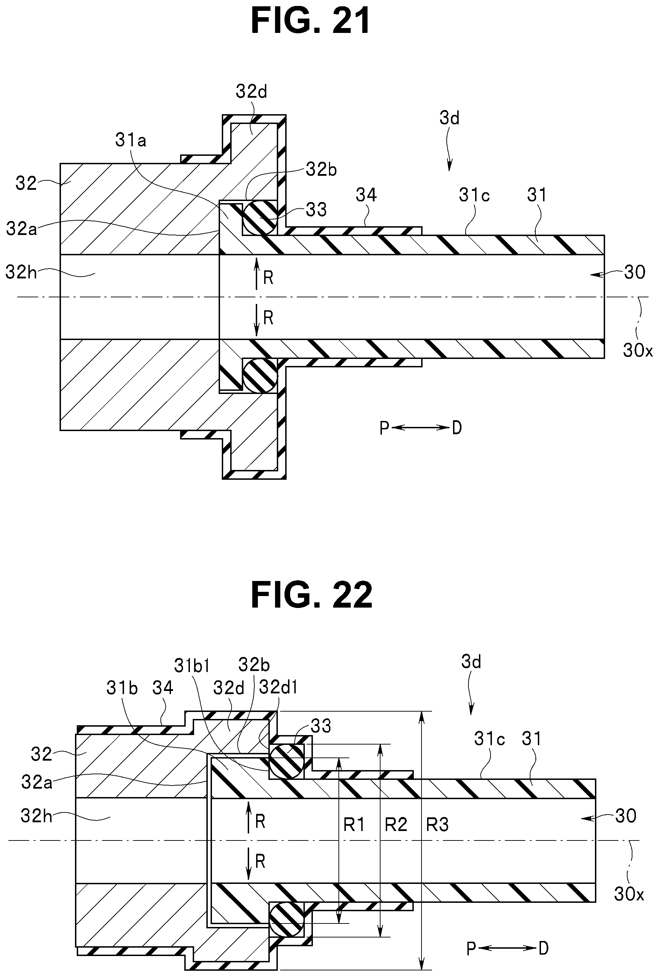

FIG. 21 is a cross-sectional view showing a fourth connecting structure of the tube 31 and the rigid member 32 in the related technology. In the fourth connecting structure, the same components as those in the first to third connecting structures are attached with the same reference signs and descriptions thereof will be omitted as appropriate. In the fourth connecting structure, description will be made mainly on points different from the first to third connecting structures.

The tube 31 includes, on the proximal end side thereof, a flange 31a (second flange) protruding in the radially outward direction R with the center axis 30x of the path 30 as a center. In the distal end of the through hole 32h of the rigid member 32, the enlarged-diameter portion 32b is provided. In the enlarged-diameter portion 32b, the receiving surface 32a, which functions as the surface for receiving the flange 31a, is provided. The flange 31a abuts against the enlarged-diameter portion 32b.

On the distal end side of the rigid member 32, a flange 32d (first flange) protruding in the radially outward direction R is formed.

The O-ring 33 (first O-ring) is assembled to the outer circumferential surface 31c of the tube 31 at a position which is more distal end side than the flange 31a, so as to abut against the flange 31a.

The proximal end side of the tube 31 including the flange 31a is inserted until the proximal end side reaches the receiving surface 32a of the rigid member 32. At this time, the O-ring 33 is arranged in the enlarged-diameter portion 32b, together with the flange 31a. The O-ring 33 is in contact with the enlarged-diameter portion 32b at a position on the inner circumferential side of the flange 32d and the flange 31a.

In the state where the heat-shrinkable tube 34 covers, from the outer circumferential surface of the rigid member 32, which is more proximal end side than the flange 32d, the flange 32d, the O-ring 33, and the proximal end side of the tube 31, heat is applied to the heat-shrinkable tube 34 to cause the heat-shrinkable tube 34 to shrink.

The fourth connecting structure provides substantially the same effects as those of the first to third connecting structures.

In addition, according to the fourth connecting structure, providing the flange 31a on the proximal end side of the tube 31 and the flange 32d on the distal end side of the rigid member 32 enables a high pulling-out strength to be ensured.

FIG. 22 is a cross-sectional view showing a fifth connecting structure of the tube 31 and the rigid member 32 in the related technology. In the fifth connecting structure, the same components as those in the first to fourth connecting structures are attached with the same reference signs and descriptions thereof will be omitted as appropriate. In the fifth connecting structure, description will be made mainly on points different from the first to fourth connecting structures.

The tube 31 includes, on the proximal end side thereof, a flange 31b (second flange) protruding in the radially outward direction R. In the distal end of the through hole 32h of the rigid member 32, the enlarged-diameter portion 32b is provided. In the enlarged-diameter portion 32b, the receiving surface 32a, which functions as the surface for receiving the flange 31b, is provided. The flange 31b abuts against the enlarged-diameter portion 32b.

The rigid member 32 includes, on the distal end side thereof, a flange 32d (first flange) protruding in the radially outward direction R.

An O-ring 33 (first O-ring) is assembled to the outer circumferential surface 31c of the tube 31 at a position which is more distal end side than the flange 31b, so as to abut against the flange 31b.

The proximal end side of the tube 31 including the flange 31b is inserted until the proximal end side reaches the receiving surface 32a of the rigid member 32. At this time, an outer diameter R2 of the O-ring 33 is larger than a diameter of the enlarged-diameter portion 32b. Therefore, the O-ring 33 is not housed in the enlarged-diameter portion 32b.

A length of the enlarged-diameter portion 32b in the direction of the center axis 30x is basically the same as a length of the flange 31b in the direction of the center axis 30x. Thus, the O-ring 33 is in contact with a surface 32d1 on the distal end side of the flange 32d and a surface 31b1 on the distal end side of the flange 31b.

In the state where the heat-shrinkable tube 34 covers, from the outer circumferential surface of the rigid member 32, which is more proximal end side than the flange 32d, the flange 32d, the O-ring 33, and the proximal end side of the tube 31, heat is applied to the heat-shrinkable tube 34 to cause the heat-shrinkable tube 34 to shrink.

The heat-shrinkable tube 34 is in contact with the O-ring 33 and is shrunk in the state of covering the O-ring 33, to compress the O-ring 33 in the direction toward the center axis 30x of the path 30.

The relationship among an outer diameter R1 of the flange 31b, the outer diameter R2 of the O-ring 33, and an outer diameter R3 of the flange 32d including the thickness of the heat-shrinkable tube 34, after the heat shrinking of the heat-shrinkable tube 34, is R1 > R2 > R3.

The fifth connecting structure provides substantially the same effects as those of the fourth connecting structure.

According to the first to fifth connecting structures, connecting structure of the tube 31 and the rigid member 32 is simplified, which facilitates the connecting work. In addition, due to the smaller number of components, the connection of the tube 31 and the rigid member 32 can be achieved at low costs. Furthermore, the O-rings 33, 33a, and 33b are used, which enables the water-tightness of the connecting part between the tube 31 and the rigid member 32 to be ensured.

In addition, using the heat-shrinkable tube 34 with the adhesive being applied, in advance, to the inner circumferential surface thereof, for example, eliminates the need of the work for applying an adhesive. Such a configuration reduces the working man-hours, thereby reducing variations of products due to variations in the work.

In addition, ensuring the water-tightness is performed with the O-rings 33, 33a, and 33b, and ensuring the connection strength is performed with the heat-shrinkable tube 34, which enables the functions of resistance to be shared by the plurality of components.

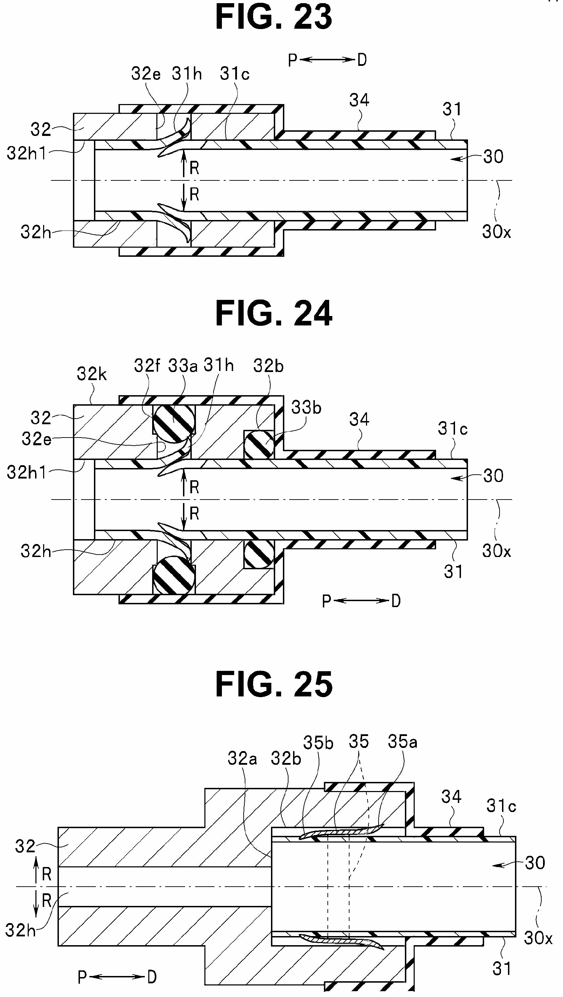

FIG. 23 is a cross-sectional view showing a sixth connecting structure of the tube 31 and the rigid member 32 in the related technology. In the sixth connecting structure, the same components as those in the first to fifth connecting structures are attached with the same reference signs and descriptions thereof will be omitted as appropriate. In the sixth connecting structure, description will be made mainly on points different from the first to fifth connecting structures.

The through hole 32h of the rigid member 32 includes, at the halfway portion thereof, a first recessed portion 32e. The first recessed portion 32e is a recessed portion which is from an inner surface 32h1 of the through hole 32h toward the radially outward direction R. In manufacturing steps, it is easier to form a hole penetrating through to the outer surface of the rigid member 32 (that is, not a bottomed hole) than to form a bottomed recessed portion from the inner surface 32h1 of the through hole 32h toward the radially outward direction R. Therefore, in the example shown in FIG. 23, the first recessed portion 32e is supposed to be a hole penetrating through to the outer surface. However, the first recessed portion 32e is not limited to the example, and may be a bottomed recessed portion.

The first recessed portion 32e may be provided at one or more locations, or two or more locations around the center axis 30x of the path 30. Therefore, if a cross section of the rigid member 32 including the center axis 30x is viewed in the part where the first recessed portion 32e is not provided, the cross section includes no hole corresponding to the first recessed portion 32e, and the thickness of the rigid member 32 is as-is present in the cross section.

A first hook 31h is provided so as to protrude from the outer circumferential surface 31c of the tube 31. One or more, or two or more first hooks 31h may be provided around the center axis 30x of the path 30 so as to correspond respectively to the first recessed portions 32e provided at one or more locations.

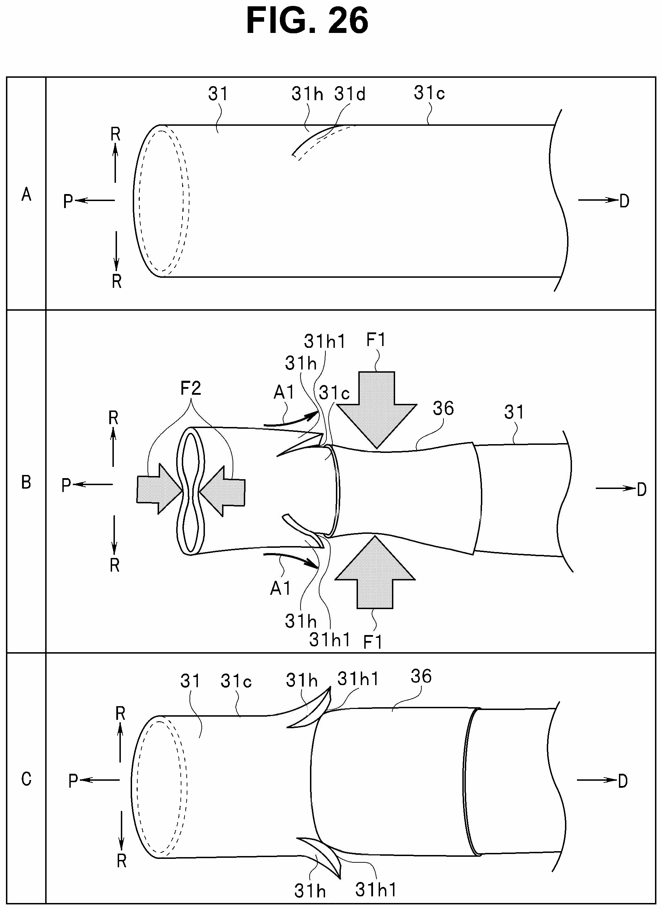

As shown in the column A in FIG. 26 to be described later, for example, the first hook 31h is formed by forming a cut 31d (see the column A in FIG. 26) and erecting the cut part in the radially outward direction R. The cut 31d is inclined with respect to the center axis 30x of the path 30 (cut diagonally toward the proximal end side (proximal end direction P side) of the tube 31). Thus, the first hook 31h is formed integrally with the tube 31, for example. Note that the example has been shown here in which the cut 31d is formed diagonally, but the cut 31d does not have to be diagonally cut.

The first hook 31h, which is in the erected state, is caught by the first recessed portion 32e and arranged in the first recessed portion 32e. The first hook 31h is caught by the first recessed portion 32e, thereby preventing the tube 31 from coming off from the rigid member 32. Therefore, the first hook 31h functions as a non-return shape and a coming-off stopper shape.

As described above, the tube 31 is formed of a resin, for example. In the configuration in which the first hook 31h provided on the tube 31 is used to prevent the tube 31 from coming off from the rigid member 32, the tube 31 having a softness whose value is approximately 70 points (A hardness is approximately 70) when the hardness is measured with Shore A (durometer A) may be used. In addition, in order to strengthen the coming-off prevention of the tube 31 from the rigid member 32, the thickness of the tube 31 may be increased.

The heat-shrinkable tube 34 is arranged on the outer circumferential sides of the tube 31 and the rigid member 32 (the part including the first recessed portion 32e). The heat-shrinkable tube 34 is in contact with the rigid member 32 and the tube 31, and is shrunk in the state of covering the rigid member 32 and the tube 31. As described above, the heat-shrinkable tube 34 with the adhesive being applied, in advance, to an inner circumferential surface thereof is used.

The work of connecting the tube 31 to the rigid member 32 is performed by the following steps (1) to (4), for example.
(1) The cut 31d is formed on the tube 31 to provide the first hook 31h.
(2) The heat-shrinkable tube 34 is inserted into the tube 31.
(3) The proximal end of the tube 31 is inserted into the rigid member 32, to cause the first hook 31h to be caught by the first recessed portion 32e.
(4) In the state where the heat-shrinkable tube 34 covers from the proximal end side than the first recessed portion 32e of the rigid member 32 to the proximal end side of the tube 31, heat is applied to the heat-shrinkable tube 34 to cause the heat-shrinkable tube 34 to shrink.

In such a configuration, the water-tightness of the connecting part between the tube 31 and the rigid member 32 is secured by the heat-shrinkable tube 34 with the adhesive being applied to the inner surface thereof. In addition, the connection strength of the connecting part between the tube 31 and the rigid member 32 is secured by the first hook 31h provided on the tube 31 being caught by the first recessed portion 32e. In other words, the water-tightness and the connection strength are achieved by the different parts.

According to the sixth connecting structure, the water-tightness and the connection strength of the connecting part between the tube 31 and the rigid member 32 can be secured. At this time, the water-tightness and the connection strength can be secured by combination of the tube 31 and the heat-shrinkable tube 34, without using an O-ring.

Furthermore, according to the sixth connecting structure, the connecting structure of the tube 31 and the rigid member 32 is simplified, which facilitates the connecting work. In addition, due to the smaller number of components, the tube 31 and the rigid member 32 can be connected to each other at a low cost.

Using the heat-shrinkable tube 34 with the adhesive being applied, in advance, to the inner circumferential surface thereof eliminates the need of the work for applying an adhesive. Such a configuration reduces the working man-hours, thereby reducing variations of products due to variations in the work.

FIG. 24 is a cross-sectional view showing a seventh connecting structure of the tube 31 and the rigid member 32 in the related technology. In the seventh connecting structure, the same components as those in the first to sixth connecting structures are attached with the same reference signs and descriptions thereof will be omitted as appropriate. In the seventh connecting structure, description will be made mainly on points different from the first to sixth connecting structures.

A second recessed portion 32f is provided on the outer circumferential surface 32k of the rigid member 32 over the entire circumference thereof. A range in which the second recessed portion 32f is provided in the direction of the center axis 30x overlaps a range in which the first recessed portion 32e is provided in the direction of the center axis 30x. The center position of the range of the second recessed portion 32f in the direction of the center axis 30x may coincide with the center position of the range of the first recessed portion 32e in the direction of the center axis 30x. Furthermore, a total value of the depth of the first recessed portion 32e and the depth of the second recessed portion 32f in the thickness direction (direction parallel to the radially outward direction R) of the rigid member 32 coincides with the thickness of the rigid member 32.

Therefore, the second recessed portion 32f and the first recessed portion 32e communicate with each other. Then, the through hole 32h communicates with the outer circumferential surface 32k side of the rigid member 32, via the first recessed portion 32e and the second recessed portion 32f.

The first O-ring 33a is arranged in the second recessed portion 32f. The first O-ring 33a water-tightly blocks a hole through which the second recessed portion 32f communicates with the first recessed portion 32e.

In addition, the through hole 32h includes, in the opening portion on the distal end thereof, the enlarged-diameter portion 32b. In the enlarged-diameter portion 32b, the second O-ring 33b externally fitted to the tube 31 is arranged. The second O-ring 33b is in contact with the rigid member 32 and an outer circumferential surface 31c of the tube 31.

The heat-shrinkable tube 34 is arranged so as to cover the outer surfaces of the tube 31, the rigid member 32, the first O-ring 33a, and the second O-ring 33b, and is shrunk. Thus, the heat-shrinkable tube 34 covers the second recessed portion 32f in which the first O-ring 33a is arranged.

According to the seventh connecting structure, the water-tightness and the connection strength can be ensured, similarly to the sixth connecting structure. Furthermore, according to the seventh connecting structure, the first O-ring 33a and the second O-ring 33b are used supplementarily, which can achieve the water-tightness higher than that in the sixth connecting structure.

FIG. 25 is a cross-sectional view showing an eighth connecting structure of the tube 31 and the rigid member 32 in the related technology. In the eighth connecting structure, the same components as those in the first to seventh connecting structures are attached with the same reference signs and descriptions thereof will be omitted as appropriate. In the eighth connecting structure, description will be made mainly on points different from the first to seventh connecting structures.

The through hole 32h includes, in the opening portion on the distal end thereof, the enlarged-diameter portion 32b. In the enlarged-diameter portion 32b, the receiving surface 32a configured to receive the proximal end of the tube 31 that is inserted into the rigid member 32 is formed. The proximal end side of the tube 31 is arranged in the enlarged-diameter portion 32b. The inner diameter of the enlarged-diameter portion 32b is larger than the outer diameter of the tube 31, and a space is created between the enlarged-diameter portion 32b and the tube 31.

In the space between the enlarged-diameter portion 32b and the tube 31, a ring member 35 that circles around the center axis 30x is arranged. The ring member 35 is formed of a material which is more rigid than the rigid member 32 and the tube 31, for example, a metal.

The ring member 35 includes first hooks 35a that are caught by the enlarged-diameter portion 32b and second hooks 35b that are caught by the tube 31. One or more sets, or two or more sets of the first hooks 35a and the second hooks 35b may be provided around the center axis 30x.

The heat-shrinkable tube 34 is in contact with the rigid member 32 and the tube 31, and is shrunk in the state of covering the rigid member 32 and the tube 31. Therefore, the space between the enlarged-diameter portion 32b in which the ring member 35 is arranged and the tube 31 is also water-tightly blocked from outside by the heat-shrinkable tube 34.

According to the eighth connecting structure, using the ring member 35, which is provided as a member separately from the tube 31 and the rigid member 32, enables the connection strength to be secured, similarly to the sixth and seventh connecting structures. In addition, using the ring member 35 can eliminate the manufacturing step of processing the tube 31.

FIG. 26 is a chart describing a manufacturing method of the tube 31 according to a ninth connecting structure of the tube 31 and the rigid member 32 in the related technology. In the ninth connecting structure, the same components as those in the first to eighth connecting structures are attached with the same reference signs and descriptions thereof will be omitted as appropriate. In the ninth connecting structure, description will be made mainly on points different from the first to eighth connecting structures.

As shown in the column A in FIG. 26, the cut 31d is made on the tube 31. The cut 31d is, for example, inclined with respect to the center axis 30x of the path 30 (cut diagonally toward the proximal end side of the tube 31). With the cut 31d, the first hook 31h is formed on the tube 31. Hereinafter, an example will be described, in which a pair of cuts 31d provided respectively at the upper and lower positions, thereby providing a pair of upper and lower first hooks 31h.

As shown in the column B in FIG. 26, a ring member 36 is arranged on the outer circumferential surface 31c of the tube 31, at a position on the more distal end side (the distal end direction D side) than the first hooks 31h. Then, a compression force from above and below (compression force applied from above and below toward the center axis 30x) is applied, as shown by the arrows F1, to the part of the tube 31, which is more distal end side than the first hooks 31h, and a compression force from left side and right side (compression force applied from left side and right side toward the center axis 30x) is applied, as shown by the arrows F2, to the part of the tube 31, which is more proximal end side (the proximal end direction P side) than the first hooks 31h. Then, as shown by the arrows A1, the upper first hook 31h erects in the upward direction (radially outward direction R) from the outer circumferential surface 31c, and the lower first hook 31h erects in the downward direction (radially outward direction R) from the outer circumferential surface 31c.

In this state, the ring member 36 is moved to the proximal end side (proximal end direction P side), to cause the ring member 36 to contact the inner surfaces 31h1 of the first hooks 31h. This maintains the pair of upper and lower first hooks 31h in the state erected from the outer circumferential surface 31c of the tube 31. Depending on how deep the ring member 36 is brought into contact with the inner surfaces 31h1 of the first hooks 31h, the degree of the erection of the first hooks 31h can be adjusted to some extent.

Applying the tube 31 in the ninth connecting structure in which the first hooks 31h are erected by using the ring member 36 as shown in FIG. 26 to the configuration shown in FIG. 23 or FIG. 24 can surely prevent the tube 31 from coming off from the rigid member 32. Thus, according to the ninth connecting structure, the connection strength of the tube 31 and the rigid member 32 can be more increased.

Furthermore, adjusting the contacting state between the ring member 36 and the inner surfaces 31h1 of the first hooks 31h enables the connection strength to be adjusted.

### [Notes]

According to the description related to the embodiments of the present disclosure, the following configurations can be obtained.

### [Note A1]

An endoscope including
a flexible tube,
the flexible tube being a tube that is along a center axis extending from one end to another end,
the flexible tube including:
   a plurality of protruded surfaces provided on an outer circumferential surface of the flexible tube; and
   recessed surfaces provided on the outer circumferential surface, the recessed surfaces being defined by the plurality of protruded surfaces, wherein
the plurality of protruded surfaces are each surrounded by the recessed surfaces, so as to be isolated from one another, and
the recessed surfaces are all inclined with respect to an axial direction parallel to the center axis, and inclined with respect to a circumferential direction around the center axis.

### [Note A2]

The endoscope according to Note A1, wherein each of the plurality of protruded surfaces forms a quadrangle when the outer circumferential surface is planarly developed.

### [Note A3]

The endoscope according to Note A1, wherein each of the plurality of protruded surfaces forms a rhombus when the outer circumferential surface is planarly developed.

### [Note A4]

The endoscope according to Note A1, wherein each of the plurality of protruded surfaces forms a rectangle when the outer circumferential surface is planarly developed.

### [Note A5]

A flexible tube that is a tube along a center axis extending from one end to another end, the flexible tube including:
a plurality of protruded surfaces provided on an outer circumferential surface of the flexible tube; and
recessed surfaces provided on the outer circumferential surface, the recessed surfaces being defined by the plurality of protruded surfaces, wherein
the plurality of protruded surfaces are each surrounded by the recessed surfaces, so as to be isolated from one another,
when the recessed surfaces each include a first part along an axial direction parallel to the center axis, the first parts are formed intermittently in the axial direction, and
when the recessed surfaces each include a second part along a circumferential direction around the center axis, the second parts are formed intermittently in the circumferential direction.

### [Note B1]

An endoscope including;
a rigid member including a through hole, the through hole constituting a part of a path;
a tube, a proximal end side of which is arranged in the through hole and an inner part of which constitutes another part of the path;
a first O-ring that is in contact with the rigid member and the tube; and
a heat-shrinkable tube that is in contact with the rigid member and the tube, and is shrunk in a state of covering the rigid member and the tube.

### [Note B2]

The endoscope according to Note B1, further including,
an enlarged-diameter portion provided at a distal end of the through hole, wherein
the first O-ring is arranged in the enlarged-diameter portion, and is in contact with an outer circumferential surface of the tube.

### [Note B3]

The endoscope according to Note B1, further including,
a second O-ring arranged on a more distal end side than the first O-ring, the second O-ring being in contact with an outer circumferential surface of the tube, wherein
the heat-shrinkable tube is further in contact with the second O-ring and is shrunk in a state of covering the second O-ring, to compress the second O-ring in a direction toward a center axis of the path.

### [Note B4]

The endoscope according to Note B1, further including,
a second O-ring which is in contact with an outer circumferential surface of the heat-shrinkable tube covering the tube.

### [Note B5]

The endoscope according to Note B1, further including:
a first flange provided on a distal end side of the rigid member;
an enlarged-diameter portion provided at a distal end of the through hole; and
a second flange which is provided on a proximal end side of the tube, and which abuts against the enlarged-diameter portion, wherein
the first O-ring is arranged in the enlarged-diameter portion, and is in contact with the enlarged-diameter portion at a position on an inner circumferential side of the first flange, and with the second flange.

### [Note B6]

The endoscope according to Note B1, further including:
a first flange provided on a distal end side of the rigid member;
an enlarged-diameter portion provided at a distal end of the through hole; and
a second flange which is provided on a proximal end side of the tube, and which abuts against the enlarged-diameter portion, wherein
the first O-ring is in contact with a surface on a distal end side of the first flange and a surface on a distal end side of the second flange, and
the heat-shrinkable tube is further in contact with the first O-ring and is shrunk in a state of covering the first O-ring, to compress the first O-ring in a direction toward a center axis of the path.

### [Note C1]

An endoscope including:
a rigid member including a through hole, the through hole constituting a part of a path;
a tube, a proximal end side of which is arranged in the through hole and an inner part of which constitutes another part of the path;
a first recessed portion extending from an inner surface of the through hole toward a radially outward direction, the first recessed portion being provided at a halfway position of the through hole of the rigid member at one or more locations around a center axis of the path;
a first hook formed so as to protrude from an outer circumferential surface of the tube, the first hook being caught by the first recessed portion to be arranged in the first recessed portion; and
a heat-shrinkable tube that is in contact with the rigid member and the tube, and is shrunk in a state of covering the rigid member and the tube.

### [Note C2]

The endoscope according to Note C1, wherein
the first hook which is configured by forming, on the tube, a cut inclined with respect to the center axis of the path, and by erecting a cut part toward the radially outward direction, the first hook being formed integrally with the tube.

### [Note C3]

The endoscope according to Note C1, further including:
a second recessed portion provided on an outer circumferential surface of the rigid member over an entire circumference, and communicating with the first recessed portion; and
an O-ring arranged in the second recessed portion, wherein
the heat-shrinkable tube further covers the second recessed portion in which the O-ring is arranged.

### [Note C4]

The endoscope according to Note C1, further including,
a ring member arranged on an outer circumferential surface of the tube, the ring member being in contact with an inner surface of the first hook, to cause the first hook to erect from the outer circumferential surface of the tube.

### [Note D1]

An endoscope including:
a rigid member including a through hole, the through hole constituting a part of a path, the rigid member including an enlarged-diameter portion provided at a distal end of the through hole;
a tube, a proximal end side of which is arranged in the enlarged-diameter portion and an inner part of which constitutes another part of the path;
a ring member formed of a material which is more rigid than the rigid member and the tube, and arranged between the enlarged-diameter portion and the tube; and
a heat-shrinkable tube that is in contact with the rigid member and the tube, and is shrunk in a state of covering the rigid member and the tube, wherein
the ring member includes a first hook that is caught by the enlarged-diameter portion and a second hook that is caught by the tube.

Note that the present disclosure is not limited as-is to the above-described embodiments. It is possible to embody the present disclosure by modifying the constituent elements in a range without departing from the gist of the disclosure at the practical stage. In addition, various aspects of the disclosure can be achieved by appropriately combining the plurality of constituent elements disclosed in the above-described embodiments. Some of the constituent elements may be deleted from all the constituent elements shown in the embodiments, for example. Furthermore, constituent elements over different embodiments may be combined as appropriate. Thus, it goes without saying that various modifications and applications can be implemented within a range without departing from the gist of the disclosure.

The present application is filed claiming the benefit of priority to U.S. provisional Application No. 63/599292 filed on November 15, 2023, the contents of which are incorporated by this reference in the description, claims, and drawings of this application.

## Claims

1. An endoscope comprising:
a flexible tube,
the flexible tube being a tube that is along a center axis extending from one end to another end,
the flexible tube including:
a plurality of protruded surfaces provided on an outer circumferential surface of the flexible tube; and
recessed surfaces provided on the outer circumferential surface, the recessed surfaces being defined by the plurality of protruded surfaces, wherein
the plurality of protruded surfaces are each surrounded by the recessed surfaces, so as to be isolated from one another,
when the recessed surfaces each include a first part along an axial direction parallel to the center axis, the first parts are formed intermittently in the axial direction, and
when the recessed surfaces each include a second part along a circumferential direction around the center axis, the second parts are formed intermittently in the circumferential direction.

2. The endoscope according to claim 1, wherein each of the plurality of protruded surfaces has a same shape.

3. The endoscope according to claim 2, wherein
each of the plurality of protruded surfaces forms a polygon when the outer circumferential surface is planarly developed.

4. The endoscope according to claim 2, wherein
each of the plurality of protruded surfaces forms a rounded polygon when the outer circumferential surface is planarly developed.

5. The endoscope according to claim 3, wherein
the recessed surfaces each include a part inclined with respect to the axial direction, and
the part inclined forms an angle of 45 degrees or less with respect to the axial direction.

6. The endoscope according to claim 3, wherein
each of the plurality of protruded surfaces forms a quadrangle when the outer circumferential surface is planarly developed.

7. The endoscope according to claim 6, wherein
each of the plurality of protruded surfaces forms a rhombus when the outer circumferential surface is planarly developed.

8. The endoscope according to claim 6, wherein
each of the plurality of protruded surfaces forms a rectangle when the outer circumferential surface is planarly developed.

9. The endoscope according to claim 2, wherein
each of the plurality of protruded surfaces forms a T-shape when the outer circumferential surface is planarly developed.

10. The endoscope according to claim 2, wherein
each of the plurality of protruded surfaces forms an L-shape when the outer circumferential surface is planarly developed.

11. The endoscope according to claim 3, wherein
each of the plurality of protruded surfaces forms a triangle when the outer circumferential surface is planarly developed.

12. The endoscope according to claim 2, wherein
the flexible tube includes:
a tube main body formed by providing the plurality of protruded surfaces and the recessed surfaces, the tube main body being made of a material having a first Young's modulus; and
filling structure parts with which the recessed surfaces on the tube main body are respectively filled, the filling structure parts being made of a filling material having a second Young's modulus lower than the first Young's modulus.

13. The endoscope according to claim 12, wherein
the filling structure parts have different filling rates of the filling material along the axial direction.

14. The endoscope according to claim 2, wherein
distances from the recessed surfaces to the plurality of protruded surfaces differ in a radial direction with the center axis as a center.

15. The endoscope according to claim 1, further comprising
an insertion portion configured to be inserted into a subject, wherein
the flexible tube is provided in the insertion portion.

16. The endoscope according to claim 1, wherein
the plurality of protruded surfaces are arranged periodically along the outer circumferential surface.

17. The endoscope according to claim 1, wherein
the plurality of protruded surfaces are arranged aperiodically along the outer circumferential surface.

18. The endoscope according to claim 1, wherein the recessed surfaces each include at least one of the first part or the second part.

19. The endoscope according to claim 1, wherein
each of the plurality of protruded surfaces has a first shape in a first range along the axial direction, and
each of the plurality of protruded surfaces has a second shape different from the first shape in a second range along the axial direction, the second range being different from the first range.

20. An endoscope comprising:
a flexible tube,
the flexible tube being a tube that is along a center axis extending from one end to another end,
the flexible tube including:
a plurality of protruded surfaces provided on an outer circumferential surface of the flexible tube; and
recessed surfaces provided on the outer circumferential surface, the recessed surfaces being defined by the plurality of protruded surfaces, wherein
the plurality of protruded surfaces are each surrounded by the recessed surfaces, so as to be isolated from one another, and
the recessed surfaces are all inclined with respect to an axial direction parallel to the center axis, and inclined with respect to a circumferential direction around the center axis.
